(19) Europäisches Patentamt
European Patent Office
Office européen des brevets

(11) **EP 4 317 457 A1**

(12) **EUROPEAN PATENT APPLICATION**

(43) Date of publication:
**07.02.2024 Bulletin 2024/06**

(21) Application number: **22382745.2**

(22) Date of filing: **01.08.2022**

(51) International Patent Classification (IPC):
**C12Q 1/6886** (2018.01) **G01N 33/574** (2006.01)

(52) Cooperative Patent Classification (CPC):
**C12Q 1/6886; G01N 33/57419;** C12Q 2600/106;
C12Q 2600/118; C12Q 2600/158; G01N 2333/4745;
G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior De Investigaciones Científicas**
**28006 Madrid (ES)**

(72) Inventors:
• **CASAL ÁLVAREZ, José Ignacio**
**E-28040 Madrid (ES)**
• **ROBLES SEBASTIÁN, Javier**
**E-28040 Madrid (ES)**

(74) Representative: **ABG Intellectual Property Law, S.L.**
**Avenida de Burgos, 16D**
**Edificio Euromor**
**28036 Madrid (ES)**

(54) **GENE SIGNATURE FOR THE PROGNOSIS OF COLORECTAL CANCER**

(57) The invention relates to methods for determining the prognosis of a patient suffering colorectal cancer, for selecting an adjuvant chemotherapy for said patient and for selecting a patient suffering colorectal cancer to receive adjuvant combination chemotherapy. The invention also relates to a computer implemented method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy and to a computer program to implement this method.

EP 4 317 457 A1

**Description**

**FIELD OF THE INVENTION**

The present invention belongs to the technical field of diagnosis of colorectal cancer and, more in particular, to methods for the prognosis of colorectal cancer as well as methods for providing personalized medicine to patients with colorectal cancer

**BACKGROUND OF THE INVENTION**

Colorectal cancer (CRC) is considered a heterogeneous disease with different outcomes according to the molecular subtypes. This heterogeneity is reflected in differential epigenetic and genetic events as microsatellite and chromosomal instability (MSI, CIN), CpG island methylator phenotype (CIMP), P53, KRAS and BRAF mutations (among others) that lead to different pathogenesis and drug sensitivity. This heterogeneity has been addressed by implementing global gene expression classifiers. Still, given the colorectal cancer heterogeneity and the various clinical outcomes, novel and simpler predictive algorithms are necessary to facilitate clinical decision-making and individually-designed management approaches. Current pathological staging presents some predictive limitations, as a significant number of CRC patients relapse after surgical resection and are likely to develop metastasis within 5 years. Particularly necessary is the stratification of stage II and stage III patients to prevent recurrence and poor outcome and to identify those patients who would benefit more of aggressive therapies.

**SUMMARY OF THE INVENTION**

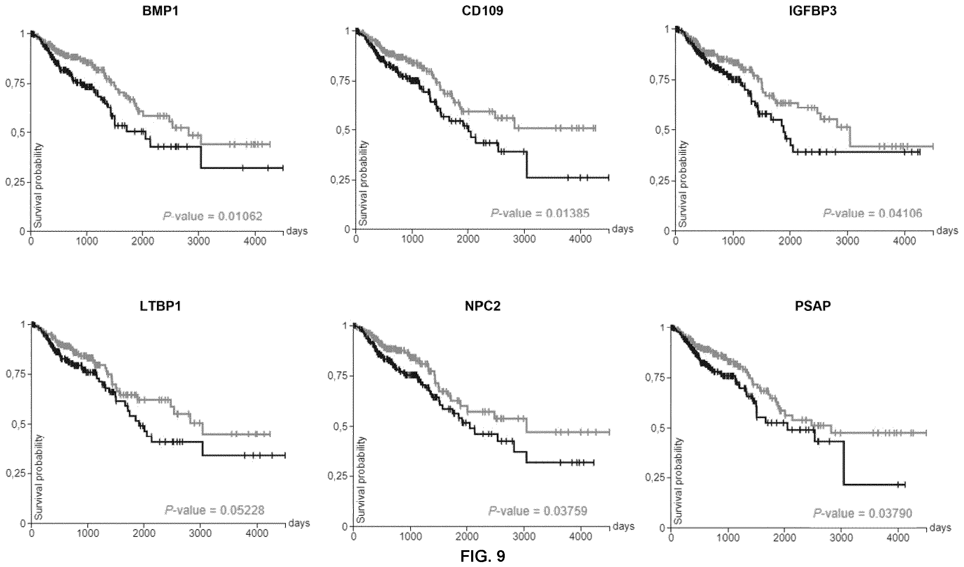

The authors of the present invention have identified genes whose expression levels provide a reliable method for the identification of patients with colorectal cancer with good or bad prognosis. For instance, Fig. 9 and Table 2 show that the expression level of any one of the genes is predictive of the prognosis of the patient. Additionally, the inventors have developed an algorithm for calculating a risk score for colorectal cancer patients based on the expression levels of these genes, confirming its predictive value in different datasets.

Therefore, in a first aspect, the invention relates to an *in vitro* method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising determining in a sample from the patient the expression level of at least one gene selected from the group consisting of CD109, PSAP, NPC2, LTBP1 and BMP1.

In another aspect, the invention relates to a computer-implemented method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising:

> (i) collecting the data of the expression level of at least one gene selected from the group consisting of CD109, PSAP, NPC2, LTBP1 and BMP1 in sample from the patient;
> (ii) analyzing the collected data by: (a) comparing the expression levels of the genes with a reference value for each gene or (b) calculating a risk score obtained by introducing the expression levels of at least three genes selected from the group consisting of CD109, PSAP, NPC2, IGFBP3, LTBP1 and BMP1 in a regression model and comparing the risk score to a reference value; and
> (iii) providing the result of the analysis.

A computer program comprising instructions in a computer readable form for implementing the computer-implemented method according to the previous aspect.

**BRIEF DESCRIPTION OF THE FIGURES**

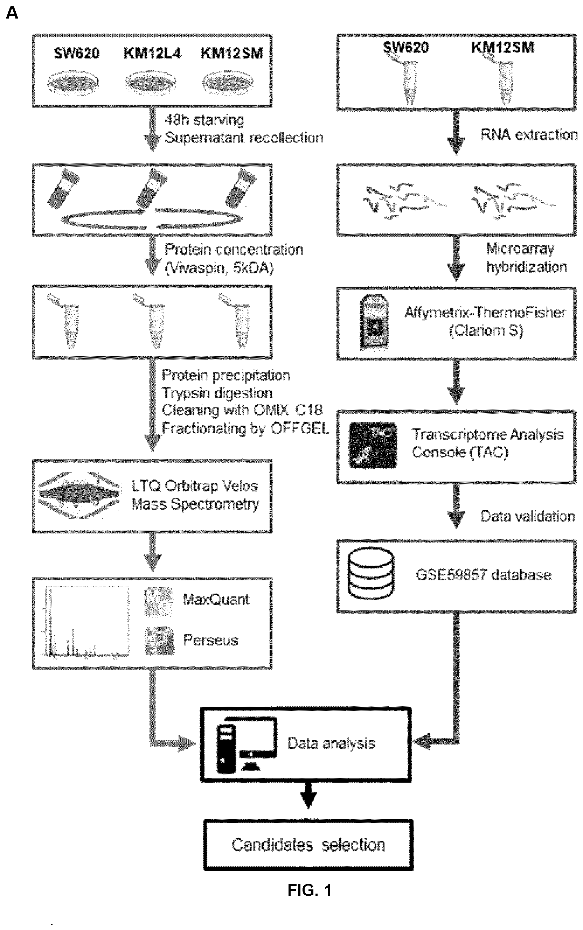

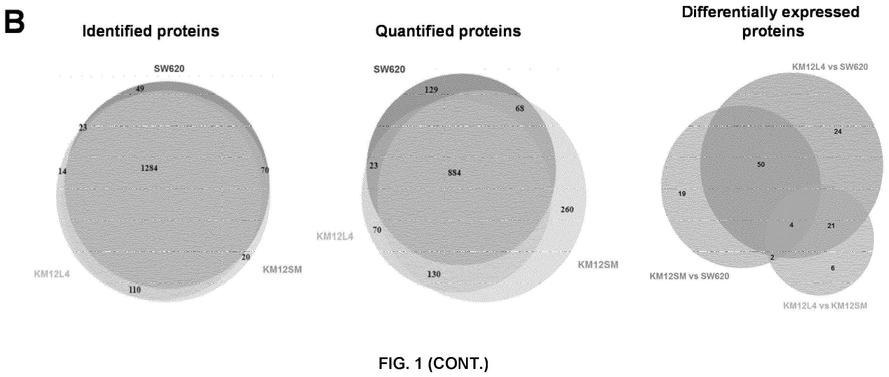

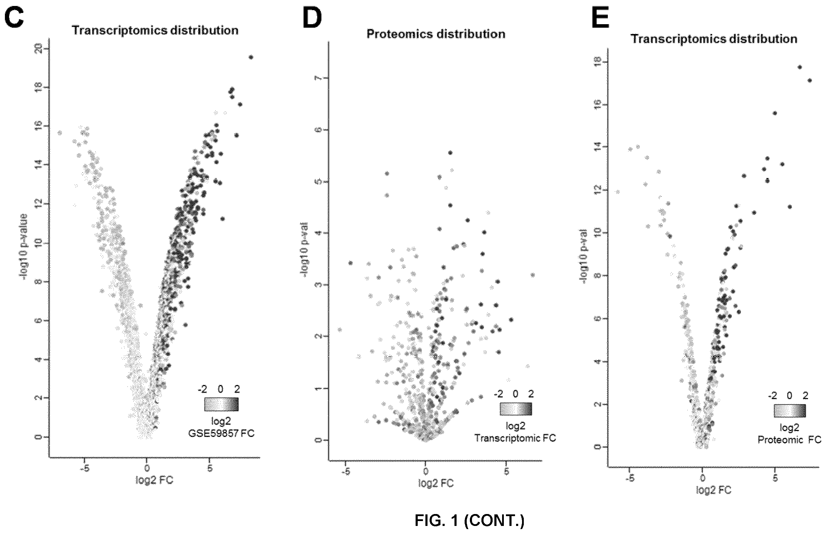

**Figure 1. Work-flow, quantified proteins and correlations between proteomic and transcriptomic results**. A) Work-flow scheme of label-free proteomic analysis of secreted proteins from SW620, KM12SM, and KM12L4 cells and the corresponding transcriptomic experiments. B) Proportional Venn diagrams of the identified, quantified and differentially-expressed proteins in the secretome fractions of the SW620, KM12SM and KM12L4 cell lines. C) Volcano plot distribution of transcriptomic data (KM12SM/SW620) according to the fold-change obtained from GSE59857 for the same cell lines. Both gene expression studies, internal and dataset, showed an excellent correlation and were used indistinctly. D) Volcano plot distributions of proteomics results colored according to transcriptomic data and E) transcriptomic results distribution represented on proteomic data.

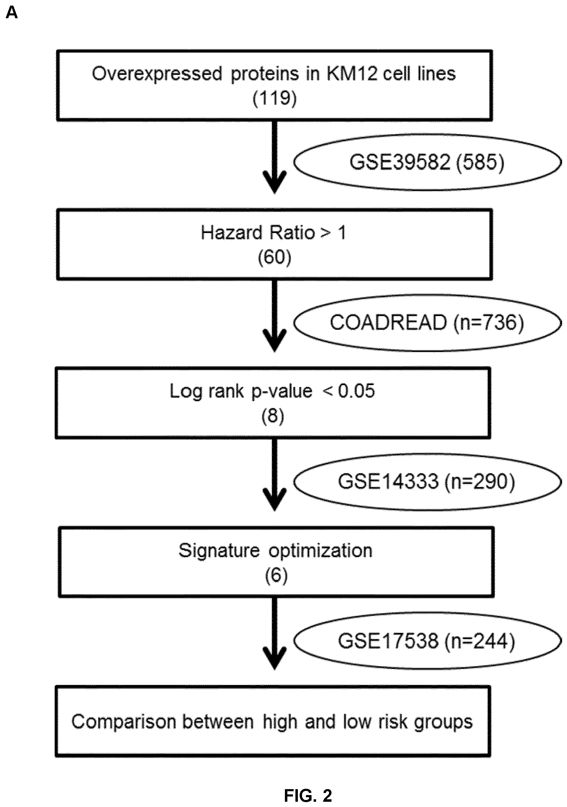

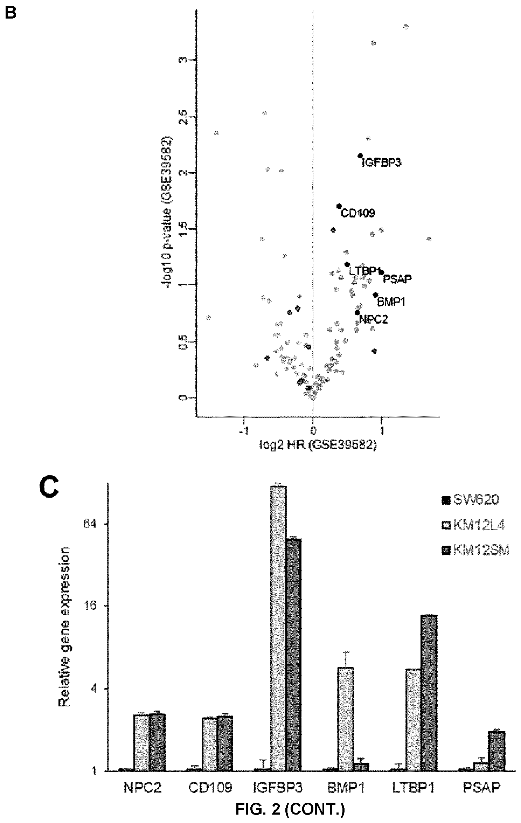

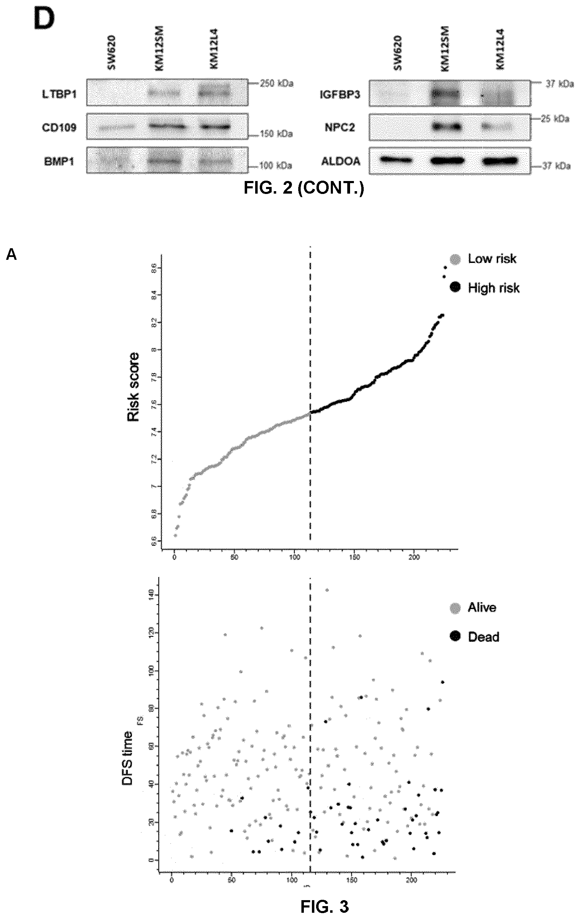

**Figure 2. Discovery and validation of the gene-based prognostic signature** A) Flowchart representation of sequential prognostic signature selection using different datasets (GSE39582, TCGA COADREAD, GSE14333 and GSE17538). B) Volcano plot of the overexpressed proteins in KM12SM and/or KM12L4 compared to SW620 cell lines. Distribution corresponds to the GSE39582 cohort data. SEC6 proteins-corresponding genes, which fit the threshold are indicated. C) Colorectal cancer cell lines KM12SM, KM12L4 and SW620 were subjected to q-PCR using specific primers for NPC2, CD109, IGFBP3, BMP1, LTBP1 and PSAP. D) Western blot analysis of the secreted fractions using specific primary antibodies. Secreted aldolase (ALDOA) was used as a loading control.

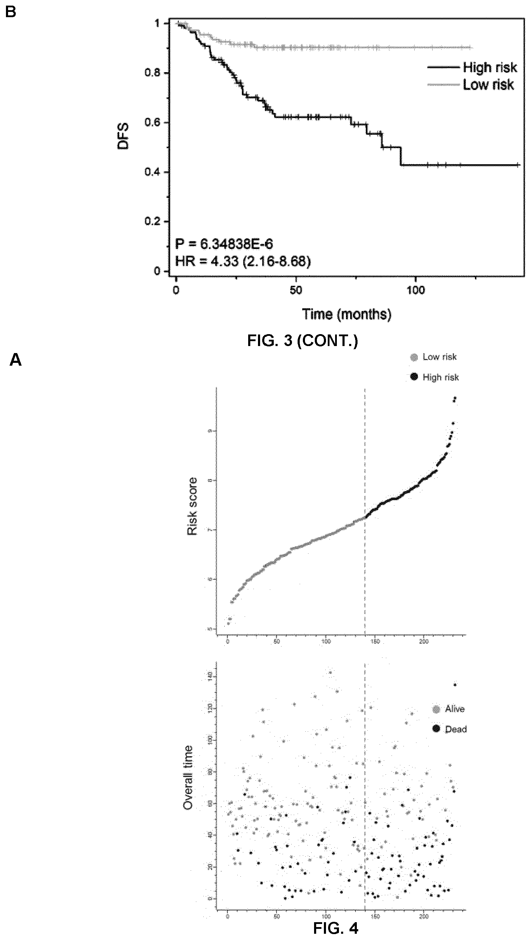

**Figure 3. Risk-score development and validation in the GSE14333 cohort.** A) Hierarchical clustering of mRNA expression in GSE17538. Overall survival event and low-high risk distribution are shown. B) Risk score distribution and corresponding survival status. B) Kaplan-Meier analysis of high and low risk patients. Hazard ratios were determined according to the Cox regression model. P values were obtained by log-rank test.

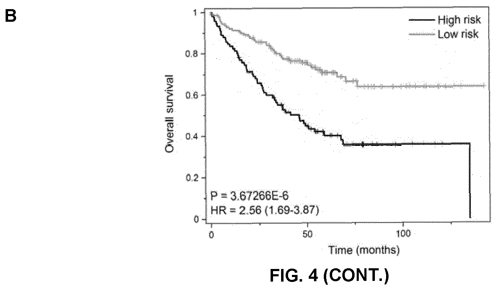

**Figure 4.** Validation of the 6-gene risk-score in GSE17538. A) Risk score distribution and corresponding survival status in GSE17538. B) Kaplan-Meier analysis of high and low risk patients.

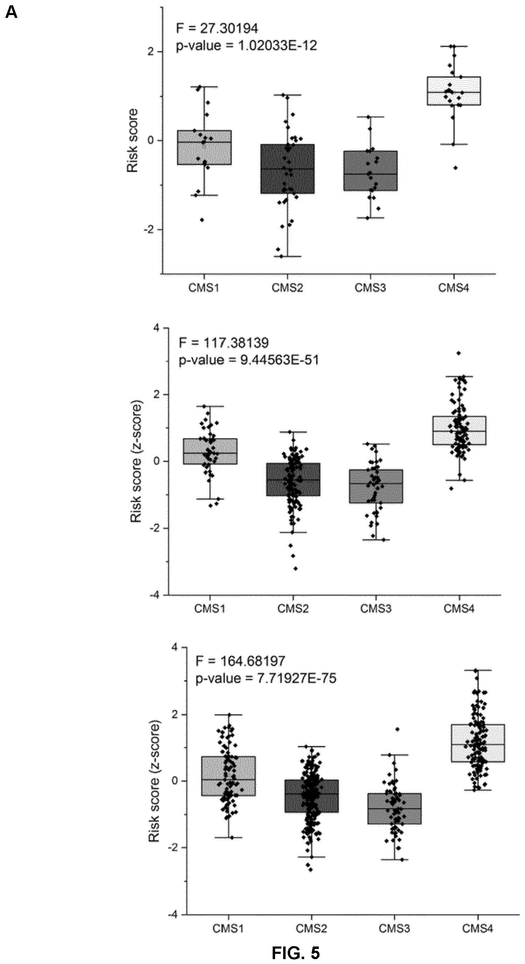

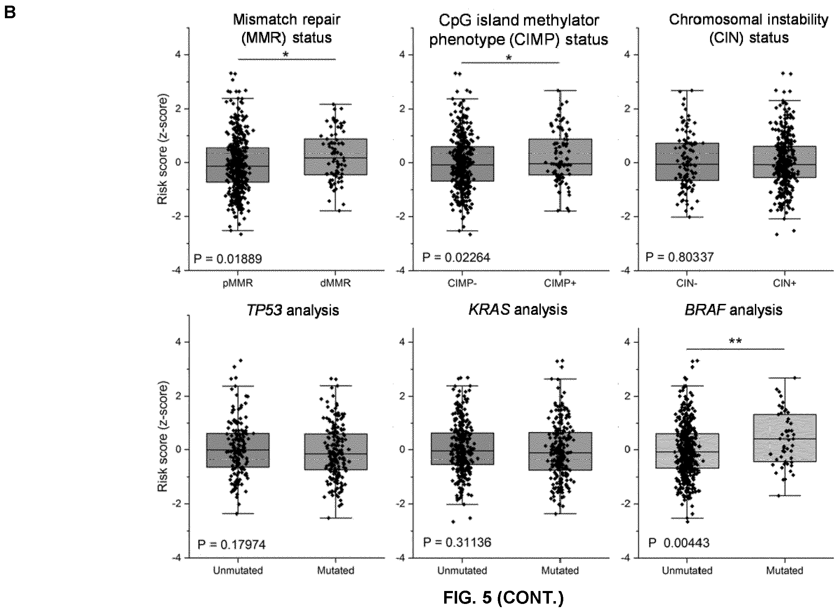

**Figure 5. Risk-score correlates with current colorectal cancer classifications.** A) SEC6 gene expression (z-score) according to the CMS classifier in GSE14333, TCGA COADREAD and GSE39582 databases. B) SEC6 expression according to pMMR and dMMR status, CIMP status, chromosomal instability and TP53, KRAS and BRAF mutations.

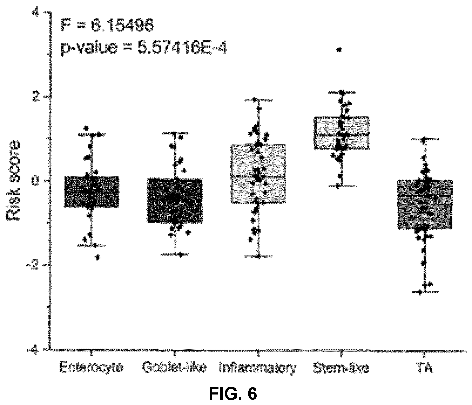

**Figure 6.** Association of risk score-based classification with current colorectal cancer classifications. A) Risk score (z-score) in Sandanandam subgroups according to GSE14333 dataset. B) High and low risk patient distribution in the CMS subgroups and vice versa. Data was obtained from GSE14333, TCGA COADREAD and GSE39582 databases.

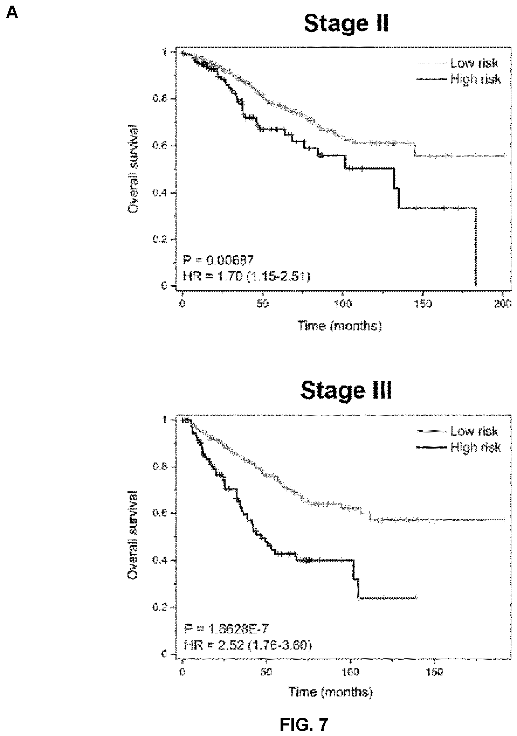

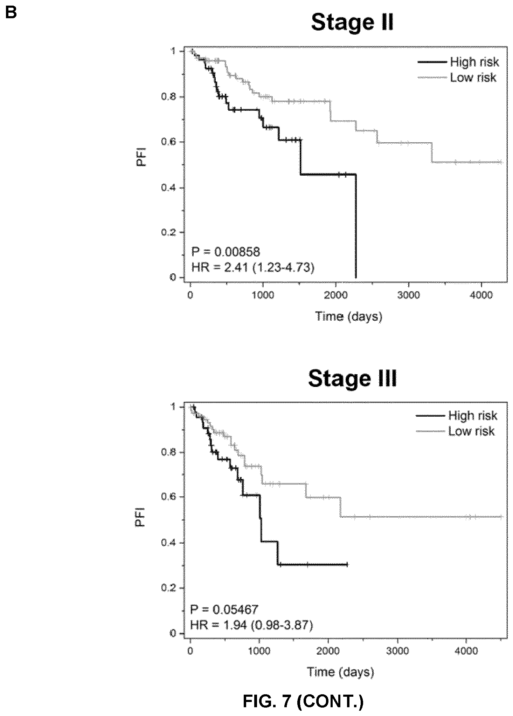

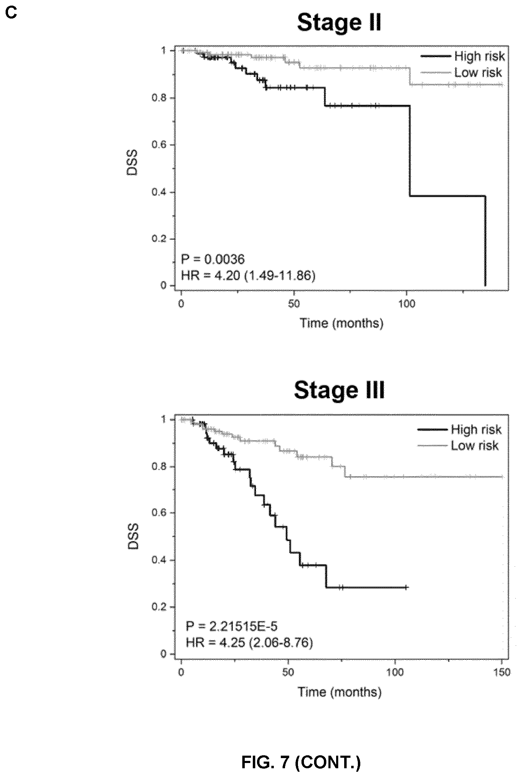

**Figure 7. High SEC6 expression is associated with lower overall survival, progression free interval and disease specific survival.** A) Overall survival (OS) analysis in the pooled cohorts GSE17538, TCGA COADREAD and GSE39582. B) Progression free Interval (PFI) analysis in the TCGA COADREAD database. C) Disease specific survival (DSS) analysis in GSE17538 and TCGA COADREAD datasets. All determinations were made for stage II and III patients using Kaplan-Meier plots. Hazard ratios were determined according to the Cox regression model. P values were obtained by log-rank test.

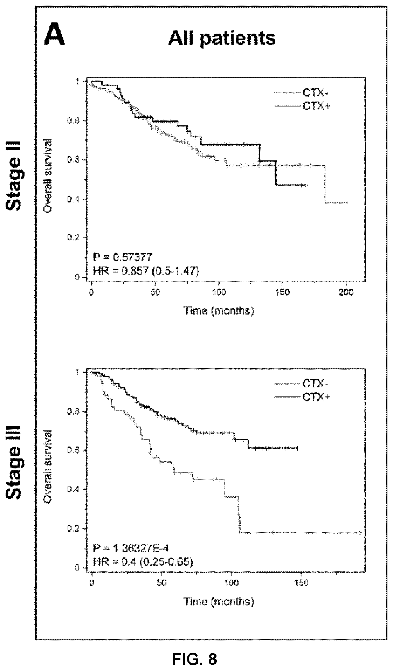

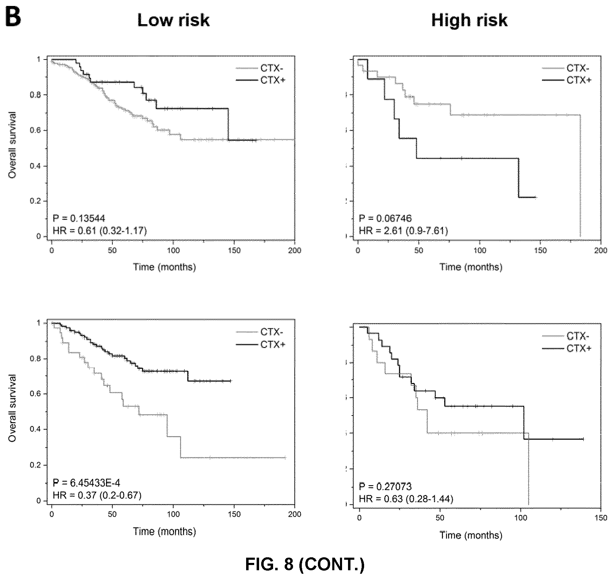

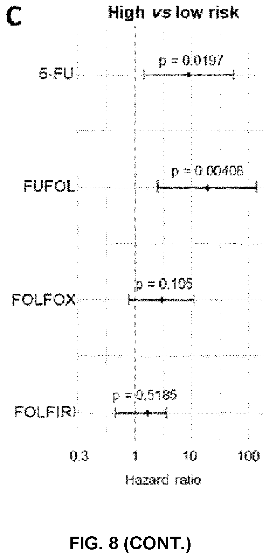

**Figure 8. SEC6-predicted high risk subgroups require more aggressive chemotherapy.** Kaplan-Meier plots of A) overall survival for all combined patients receiving 5-FU or FUFOL chemotherapy (CTX+) or not (CTX-) according to the AJCC stage (II or III) and B) overall survival for high and low risk subgroups after receiving chemotherapy (CTX+) or not (CTX-). Hazard ratios were determined according to the Cox regression model. P values were obtained by log-rank test. C) Forest plots of hazard ratios associated to each treatment. P values were obtained by Cox regression analysis. Patient data were obtained from GSE39582 (5-FU, FUFOL), GSE39582 and GSE72970 (FOLFIRI) and GSE39852, GSE72970 and GSE106584 (FOLFOX) cohorts.

**Figure 9.** Kaplan-Meier individual analysis of the 6 genes that constitute the SEC6 signature. BMP1, CD109, IGFBP3, LTBP1, NPC2 and PSAP were analysed by log rank analysis. Patients were divided in two subpopulations of the same size, high expression (black) and low expression (gray). TCGA COADREAD dataset was used for the analysis.

## DETAILED DESCRIPTION OF THE INVENTION

*In vitro methods of the invention*

In a first aspect, the invention relates to *in vitro* method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising determining in a sample from the patient the expression level of at least one gene selected from the group consisting of CD109, PSAP, NPC2, LTBP1 and BMP1.

In a particular embodiment, the method further comprises determining the expression level of IGFBP3.

The term *"in vitro",* as used herein, refers to the fact that the method is not carried out on the body of a human or animal subject, but rather on cells or fluids isolated from said subject or in a test tube.

The method of the invention allows determining the prognosis of a patient suffering colorectal cancer.

The term "prognosis" refers to a prediction of medical outcome, for example, a poor or good outcome (e.g., likelihood of long-term survival, overall survival, disease-specific survival, progression-free survival or disease-free survival); a negative prognosis, or poor outcome, includes a prediction of relapse, disease progression (e.g., tumor growth or metastasis, or drug resistance), or mortality; a positive prognosis, or good outcome, includes a prediction of disease remission, (e.g., disease-free status), amelioration (e.g., tumor regression), or stabilization.

Any parameter which is widely accepted for determining prognosis of a patient can be used in the present invention including, without limitation:

- Overall survival rate, as used herewith, relates to the percentage of people in a study or treatment group who are alive for a certain period of time after they were diagnosed with or treated for a disease, such as cancer.
- Disease-specific survival rate which is defined as the percentage of people in a study or treatment group who have not died from a specific disease in a defined period of time.
- Disease-free survival (DFS), as used herewith, is understood as the length of time after treatment for a disease during which a subject survives with no sign of the disease.
- Objective response which, as used in the present invention, describes the proportion of treated subjects in whom a complete or partial response is observed.
- Tumor control which, as used in the present invention, relates to the proportion of treated subjects in whom complete response, partial response, minor response or stable disease $\geq$ 6 months is observed.
- Progression free survival which, as used herein, is defined as the time from start of treatment to the first measurement of cancer growth.
- Time to progression (TTP), as used herein, relates to the time since a disease is treated until the disease starts to get worse. The term "progression" has been previously defined.
- Six-month progression free survival or "PFS6" rate which, as used herein, relates to the percentage of subjects who are free of progression in the first six months after the initiation of the therapy and
- Median survival which, as used herein, relates to the time at which half of the subjects enrolled in the study are still alive.
- Distant metastasis-free survival or "DMFS", as used herein, refers to the time from diagnosis and/or first surgery to treat a cancer patient to the time of first distant metastasis.
- Recurrence as used herein refers to the appearance of cancer after treatment and after a period of time during which cancer was not detected.

In a particular embodiment, the prognosis is determined as overall survival, disease-specific survival, disease-free survival, distant metastasis-free survival or recurrence.

As it will be understood by those skilled in the art, the prognosis, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, cross-validated classification rates and the like etc. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.01, 0,005 or lower.

The method of the invention also allows selecting an adjuvant chemotherapy for a patient suffering colorectal cancer.

The method of the invention also allows selecting a patient suffering colorectal to receive adjuvant combination chemotherapy.

As used herein, the terms "treatment" or "therapy" can be used indistinctly and refer to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment The term "adjuvant chemotherapy" as used herein means treatment of cancer with standard chemotherapeutic agents after surgery where all detectable disease has been removed, but where there still remains a risk of small amounts of remaining cancer. The term "chemotherapy" refers to the use of drugs to destroy cancer cells. The drugs are generally administered through oral or intravenous route. Sometimes, chemotherapy is used together with radiation treatment.

In particular embodiment, adjuvant chemotherapy is selected for a colorectal patient after the patient undergone surgical resection of the tumor.

The term "adjuvant chemotherapy", at used herein refers to a treatment of cancer with standard chemotherapeutic agents after surgery where all detectable disease has been removed, but where there still remains a risk of small amounts of remaining cancer.

The term "chemotherapy" refers to the use of drugs to destroy cancer cells. The drugs are generally administered through oral or intravenous route. Sometimes, chemotherapy is used together with radiation treatment.

Adjuvant chemotherapy can be a combination therapy or a single-agent therapy. The term "adjuvant combination chemotherapy", as used herein, refers to the combined use of more than one chemotherapeutic agent. In a particular embodiment, combination chemotherapies are combinations comprising at least two of the following chemotherapeutic agents: folinic acid, 5-fluorouracil, irinotecan and oxaliplatin. In a more particular embodiment, the adjuvant combination therapy is selected from the group consisting of: a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride and oxaliplatin (FOLFIRINOX).

The term "single-agent adjuvant chemotherapy", as used herein, refers to the use of a single chemotherapeutic agent. In a particular embodiment, the single-agent adjuvant chemotherapy is 5-fluorouracil (5FU).

The term "patient" or "subject" refers to all animals classified as mammals and includes but is not limited to domestic and farm animals, primates and humans, for example, human beings, non-human primates, cows, horses, pigs, sheep, goats, dogs, cats, or rodents. Preferably, the subject is a human man or woman of any age or race. In a preferred embodiment, the subject has not been treated with chemotherapy or radiotherapy prior to the determination of the expression levels of the gene or genes of interest. In yet another embodiment, the patient has undergone surgical resection of the tumor.

The term "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. In the staging systems used for classification of colorectal cancer, the colon and rectum are treated as one organ.

The term "colorectal cancer" includes any stage of said cancer, including stage I, stage II, stage III or stage IV tumor, wherein Stage I is defined as either T1 N0 M0 or T2 N0 M0; Stage II is defined as T3 N0 M0 or T4 N0 M0; Stage III is defined as any T, N1-2; M0 and Stage IV correspond to any T, any N, M1. According to the tumor, node, metastasis (TNM) staging system of the American Joint Committee on Cancer (AJCC) (Greene et al. (eds.), AJCC Cancer Staging Manual. 6th Ed. New York, N.Y.: Springer; 2002), the various stages of colorectal cancer are defined as follows:

- Tumor: T1: tumor invades submucosa; T2: tumor invades muscularis propria; T3: tumor invades through the muscularis propria into the subserose, or into the pericolic or perirectal tissues; T4: tumor directly invades other organs or structures, and/or perforates.
- Node: N0: no regional lymph node metastasis; N1: metastasis in 1 to 3 regional lymph nodes; N2: metastasis in 4 or more regional lymph nodes.
- Metastasis: M0: mp distant metastasis; M1: distant metastasis present.

In a particular embodiment, the patient has stage II or stage III colorectal cancer.

In a particular embodiment, the patient has been diagnosed with colorectal cancer and has had surgical resection of the cancer. In a particular embodiment, the patient has had a surgical resection of a stage I tumor, of a stage II tumor, of a stage III tumor or of a stage IV tumor, preferably a stage II or stage III tumor.

In the present invention, the term "sample" or "biological sample" means biological material isolated from a subject. The biological sample can contain any biological material suitable for detecting the expression levels of the desire genes, that is, the sample should contain mRNA and/or proteins, preferably mRNA and/or proteins from the colorectal tumor cells, and can comprise cell and/or non-cell material of the subject. In a particular embodiment, the sample is a tumor sample, circulating tumor cells or a biofluid.

The term "tumor sample" refers to a sample from the colorectal tumor, preferably obtained by biopsy.

The term "circulating tumor cells" or "CTCs" refer to tumor cells that have sloughed off the primary tumor and extravasate into and circulate in the blood.

The term "biofluid", as used herein, refers to aqueous fluids of biological origin. The biofluid may be obtained from any location (such as blood, plasma, serum, urine, bile, cerebrospinal fluid, aqueous or vitreous humour, or any bodily secretion), an exudate (such as fluid obtained from an abscess or any other site of infection or inflammation), or fluid obtained from a joint (such as a normal joint or a joint affected by disease such as rheumatoid arthritis). In a particular embodiment, the biofluid is blood, serum or plasma. In a particular embodiment, the biofluid comprises circulating tumor cells or mRNA from circulating tumor cells.

The method of the invention comprises determining the expression level of at least one gene selected from the group consisting of IGFBP3, CD109, LTBP1, PSAP, BMP1 and NPC2 in a sample from the subject.

The term "expression level", as used herein, refers to a measurable quantity of a gene product produced by the gene in a sample of the subject, wherein the gene product can be a transcriptional product or a translational product. As understood by the person skilled in the art, the gene expression level can be quantified by measuring the levels of the transcriptional product of said gene (messenger RNA when the gene is a protein-coding gene) or of the protein encoded by said gene.

The level of a messenger RNA can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA).

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labelled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labelled antibodies (primary antibodies) and labelled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays.

In a particular embodiment, the determination of the expression level of the gene or genes is carried out by determining the levels of the corresponding mRNAs or by determining the levels of the polypeptides encoded by said genes

In a particular embodiment, the expression level of the at least one gene is determined by measuring the levels of the transcriptional product of said gene (messenger RNA). In a more particular embodiment, the expression level of the at least one gene is determined by quantitative PCR (qPCR). In another particular embodiment, the expression level of the genes is determined by an oligonucleotide microarray.

The term "CD109", as used herein, refers to a gene encoding the CD109 protein. The human CD109 is assigned the Gene ID 135228 (NCBI GenBank, 3 July 2022 update). The term "CD109" includes any of the transcript variants that have been described for this gene. The protein encoded by the CD109 human gene has the amino acid sequence defined under the accession number Q6YHK3 in UniProtKB/Swiss-Prot (version 145 of the entry 2 as of 25 May 2022).

The term "LTBP1", as used herein, refers to a gene encoding the latent transforming growth factor beta binding protein 1. The human LTBP1 is assigned the Gene ID 4052 (NCBI GenBank, 19 June 2022 update). The term "LTBP1" includes any of the transcript variants that have been described for this gene. The protein encoded by the LTBP1 human gene has the amino acid sequence defined under the accession number Q14766 in UniProtKB/Swiss-Prot (version 204 of the entry 4 as of 25 May 2022).

The term "PSAP" or "GLBA" or "SAP1", as used herein, refers to a gene encoding the protein prosaposin. The human PSAP is assigned the Gene ID 5660 (NCBI GenBank, 3 July 2022 update). The term "PSAP" includes any of the transcript variants that have been described for this gene. The protein encoded by the PSAP human gene has the amino acid sequence defined under the accession number P07602 in UniProtKB/Swiss-Prot (version 250 of the entry 2 as of 25 May 2022).

The term "BMP1", as used herein, refers to a gene encoding the bone morphogenetic protein 1. The human BMP1 is assigned the Gene ID 649 (NCBI GenBank, 3 July 2022 update). The term "BMP1" includes any of the transcript variants that have been described for this gene. The protein encoded by the BMP1 human gene has the amino acid sequence defined under the accession number P13497 in UniProtKB/Swiss-Prot (version 232 of the entry 2 as of 25 May 2022).

The term "NPC2", as used herein, refers to a gene encoding protein NPC intracellular cholesterol transporter 2. The human NPC2 is assigned the Gene ID 10577 (NCBI GenBank, 3 July 2022 update). The term "BMP1" includes any of the transcript variants that have been described for this gene. The protein encoded by the BMP1 human gene has the amino acid sequence defined under the accession number P61916 in UniProtKB/Swiss-Prot (version 165 of the entry 1 as of 25 May 2022).

The term "IGFBP3", as used herein, refers to a gene encoding the protein insulin like growth factor binding protein 3. The human IGFBP3 is assigned the Gene ID 3486 (NCBI GenBank, 18 July 2022 update). The term "IGFBP3" includes any of the transcript variants that have been described for this gene. The protein encoded by the human IGFBP3 gene has the amino acid sequence defined under the accession number P17936 in UniProtKB/Swiss-Prot (version 230 of the entry 2 as of 25 May 2022).

In a particular embodiment, the method comprises determining the expression level of at least one, at least 2, at least 3, at least 4 or the 5 genes CD109, LTBP1, PSAP, BMP1 and NPC2 and, optionally, IGFBP3.

In a particular embodiment, the method comprises determining the expression level of at least CD109. In a more particular embodiment, the method comprises determining the expression level of CD109 and at least one, at least 2, at least 3, at least 4 or the other 5 genes IGFBP3, LTBP1, PSAP, BMP1 and NPC2.

In a particular embodiment, the method comprises determining the expression level of at least LTBP1. In a more particular embodiment, the method comprises determining the expression level of LTBP1 and at least one, at least 2, at least 3, at least 4 or the other 5 genes IGFBP3, CD109, PSAP, BMP1 and NPC2.

In a particular embodiment, the method comprises determining the expression level of at least PSAP. In a more particular embodiment, the method comprises determining the expression level of PSAP and at least one, at least 2, at least 3, at least 4 or the other 5 genes IGFBP3, LTBP1, CD109, BMP1 and NPC2.

In a particular embodiment, the method comprises determining the expression level of at least BMP1. In a more particular embodiment, the method comprises determining the expression level of BMP1 and at least one, at least 2, at least 3, at least 4 or the other 5 genes IGFBP3, LTBP1, PSAP, CD109 and NPC2.

In a particular embodiment, the method comprises determining the expression level of at least NPC2. In a more particular embodiment, the method comprises determining the expression level of NPC2 and at least one, at least 2, at least 3, at least 4 or the other 5 genes IGFBP3, LTBP1, PSAP, BMP1 and CD109.

In a particular embodiment, the method comprises determining the expression level of CD109 and IGFBP3, and optionally at least one, at least two, at least three or the other four genes LTBP1, PSAP, BMP1 and NPC2.

In a particular embodiment, the method comprises determining the expression level of:

- CD109 and IGFBP3.
- CD109 and LTBP1.
- CD109 and PSAP.
- CD109 and BMP1.
- CD109 and NPC2.
- CD109, IGFBP3 and LTBP1.
- CD109, IGFBP3 and PSAP.
- CD109, IGFBP3 and BMP1.
- CD109, IGFBP3 and NPC2.
- CD109, LTBP1 and PSAP.
- CD109, LTBP1 and BMP1.
- CD109, LTBP1 and NPC2.
- CD109, PSAP and BMP1.
- CD109, PSAP and NPC2.
- CD109, BMP1 and NPC2.
- CD109, IGFBP3, LTBP1 and PSAP.
- CD109, IGFBP3, LTBP1 and BMP1.
- CD109, IGFBP3, LTBP1 and NPC2.
- CD109, IGFBP3, PSAP and BMP1.
- CD109, IGFBP3, PSAP and NPC2.
- CD109, IGFBP3, BMP1 and NPC2.
- CD109, LTBP1, PSAP and BMP1.
- CD109, LTBP1, PSAP and NPC2.
- CD109, LTBP1, BMP1 and NPC2.
- CD109, PSAP, BMP1 and NPC2.
- CD109, IGFBP3, LTBP1, PSAP and BMP1.
- CD109, IGFBP3, LTBP1, PSAP and NPC2.
- CD109, IGFBP3, LTBP1, PSAP, BMP1 and NPC2.

In a particular embodiment, the method of the invention further comprises comparing the expression level of the at least one gene with a reference value, wherein

- if the expression level of the at least one gene is increased compared to the reference value, the patient has a poor prognosis, or adjuvant combination chemotherapy is selected for the patient, or the patient is selected for adjuvant combination chemotherapy, or

- if the expression level of the at least one gene is decreased compared to the reference value, the subject has a good prognosis, or single-agent adjuvant chemotherapy or no chemotherapy is selected for the patient, or the patient is selected for a single-agent adjuvant chemotherapy or for no chemotherapy.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on a large number of samples, such as from population of subjects of the chronological age matched group, or based on a pool of samples.

In a preferred embodiment, the reference value according to this embodiment of the invention is obtained from samples of colorectal cancer from one or more patients identified as patients having good prognosis, or from one or more patients identified as patients having bad prognosis, or from a pool of samples of colorectal cancer from patients having good prognosis and patients having bad prognosis. Even more preferably, the reference value is obtained from a pool of samples of colorectal cancer from patients having good prognosis and patients having bad prognosis.

Alternatively, the reference value according to this embodiment of the method of the invention could also be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment, the reference value is the expression level of the gene of interest in a non-cancerous colorectal sample. In another particular embodiment, the reference value is the expression level of the gene of interest in a sample of colorectal cancer from one or more patients identified as patients having good prognosis. In another embodiment, the reference value can correspond to an average value obtained from a pool of non-cancerous colorectal sample. Said reference sample is typically obtained by combining equal amounts of samples from a subject population.

When the expression level of the gene of interest is determined as the levels of mRNA, then the reference value should also be obtained from mRNA levels. When the expression level of the gene of interest is determined as the level of the protein encoded by said gene, then the reference value should also be obtained from protein levels.

Once the expression levels of the genes in relation to reference values for said genes have been determined, it is necessary to identify if there are alterations in the expression of said genes (increase or decrease of the expression). The expression of a gene is considered increased in a sample of the patient under study when the levels increase with respect to the reference values by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a gene is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

The term "good prognosis", as used herein, indicates that the patient is expected (e.g. predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for a heterogeneous cancer patient population. An overall "low" risk of recurrence was considered to be lower than 15 percent. The risk will also vary in function of the time period. The time period can be, for example, five years, ten years, fifteen years or even twenty years after initial diagnosis of cancer or after the prognosis is made. Colorectal cancer patients with good prognosis are selected for less aggressive adjuvant chemotherapies, such as single-agent adjuvant chemotherapy, for example, a 5FU therapy, or are selected to receive no adjuvant chemotherapy.

As used herein, "poor prognosis" indicates that the patient is expected, i.e. predicted, to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. The risk will also vary in function of the time period. The time period can be, for example, five years, ten years, fifteen years or even twenty years of initial diagnosis of cancer or after the prognosis was made. Colorectal cancer patients with bad prognosis are selected for more aggressive adjuvant chemotherapies, such as adjuvant combined chemotherapy, for example, FUFOL, FOLFIRI, FOLFOX or FOLFIRINOX.

In a particular embodiment, the method of the invention comprises determining the expression levels, preferably the levels of mRNA, of at least one, at least 2, at least 3, at least 4 or the 5 genes CD109, PSAP, NPC2, LTBP1 and BMP1 and, optionally, IGFBP3, and comparing the expression level of each gene with a reference value. In another particular embodiment, the method comprises determining the expression level, preferably the levels of mRNA, of at least CD109 and optionally at least one, at least 2, at least 3, at least 4 or the other 5 genes IGFBP3, LTBP1, PSAP, BMP1 and NPC2 and comparing the expression level of each gene with a reference value. In another particular embodiment, the method of the invention comprises determining the expression level, preferably the levels of mRNA, of any of the above mentioned combinations of the genes CD109, PSAP, NPC2, LTBP1 and BMP1 and, optionally, IGFBP3, and comparing said expression level to the reference value for each gene.

In a particular embodiment, the method of the invention further comprises (i) calculating a risk score obtained by introducing the expression level of the at least three genes selected from the group consisting of CD109, PSAP, NPC2, IGFBP3, LTBP1 and BMP1 in a regression model and (ii) comparing the risk score to a reference value, wherein

- if the risk score is increased compared to the reference value, the patient has a poor prognosis, or adjuvant combination chemotherapy is selected for the patient, or the patient is selected for adjuvant combination chemotherapy, or
- if the risk score is decreased compared to the reference value, the patient has a good prognosis, or a single-agent adjuvant chemotherapy or no adjuvant chemotherapy is selected, or the patient is selected for a single-agent adjuvant chemotherapy or for no adjuvant chemotherapy.

The term "risk score", as used herein, refers to a value assigned to a given combination of factors and which reflects the degree to which said combination of factors influences the probability of an outcome, such as the prognosis e of a patient.

In a preferred embodiment, the risk value or score is calculated from the weighted expression levels of the genes assayed, where the weighted expression levels are obtained by multiplying the expression level of each gene by a weighting factor or "weight", to arrive at weighted expression levels for each of the one or more genes.

The risk score is calculated by introducing the expression level of the at least three genes in a regression model. The term "regression model", as used herein, refers to a statistical model for estimating the relationship among parameters. For instance, a "regression model" includes a linear regression or a logistic regression that estimates a parameter for a function based on independent variables. A regression model may likewise use Stochastic Gradient Descent, Adaptive Gradient Algorithm ("AdaGrad"), Adaptive Moment Estimation ("Adam"), Alternating Direction Method of Multipliers ("ADMM"), or other optimization algorithms.

In a particular embodiment, the risk score is determined according to the formula (I)

$$risk\ score = \Sigma\ (\beta i \times Ei)$$

wherein:

βi is the regression coefficient of each gene and
Ei is the normalized expression level of each gene.

The term "regression coefficient", as used herein, refers to a parameter that describes the relationship between a predictor variable and the response. In linear regression models, coefficients are the values that multiply the predictor values.

The term "normalized expression level", as used herein, refers to a value which is obtained in a particular kind of expression measurement and which is normalized to make it comparable across experiments (e.g. normalized expression from microarrays, normalized expression from RNA-sequencing). In a particular embodiment, the expression levels of the genes are mRNA expression levels and are normalized. In a particular embodiment, the expression levels of the genes, particularly the levels or mRNA, are normalized based on the z-score method (number of standard deviations in which a given value is above or below from the mean of a certain sample or population). In another particular embodiment, the expression levels of the genes, particularly the levels or mRNA, are normalized based on the expression level of housekeeping genes to avoid the heterogeneity of the technique. In this particular embodiment, the normalized value of each of the genes (CD109, BMP1, PSAP, IGFBP3, LTBP1 and/or NPC2) is obtained by dividing the expression level of each gene by the mean of the expression levels of the housekeeping genes.

In a particular embodiment, the regression model is a Cox model. The term "Cox regression model" or "Cox Proportional Hazard model", as used herein, refers to a conventional statistical model for survival analysis. In addition to classification algorithms that treat binary or multivalued outcomes as-is, Cox regression uses predictive variables such as survival (eg, months or years) or actual periods of time without side effects or recurrence of the disease. It also defines a semiparametric model that is directly associated with outcomes. The multivariate Cox function is considered the best hazard function in terms of identifying time-to-live endpoints for combining independent parameters.

In a particular embodiment, the regression model is a Cox model and the regression coefficients of each of the genes are those included in Table 1.

Table 1. Cox regression coefficients

| Gene | Regression coefficient |
| --- | --- |
| CD109 | 0.5022 |
| IGFBP3 | 0.7401 |
| LTBP1 | 0.5903 |
| PSAP | 1.0836 |
| BMP1 | 0.7639 |
| NPC2 | 0.4 |

In a particular embodiment, the risk score is calculated based on the expression levels, preferably the levels of mRNA, of at least 3, at least 4, at least 5 or the 6 genes CD109, PSAP, NPC2, IGFBP3, LTBP1 and BMP1. In another particular embodiment, the risk score is calculated based on the expression level of any of the following combinations of the genes:

- CD109, IGFBP3 and LTBP1.
- CD109, IGFBP3 and PSAP.
- CD109, IGFBP3 and BMP1.
- CD109, IGFBP3 and NPC2.
- CD109, LTBP1 and PSAP.
- CD109, LTBP1 and BMP1.
- CD109, LTBP1 and NPC2.
- CD109, PSAP and BMP1.
- CD109, PSAP and NPC2.
- CD109, BMP1 and NPC2.
- CD109, IGFBP3, LTBP1 and PSAP.
- CD109, IGFBP3, LTBP1 and BMP1.
- CD109, IGFBP3, LTBP1 and NPC2.
- CD109, IGFBP3, PSAP and BMP1.
- CD109, IGFBP3, PSAP and NPC2.
- CD109, IGFBP3, BMP1 and NPC2.
- CD109, LTBP1, PSAP and BMP1.
- CD109, LTBP1, PSAP and NPC2.
- CD109, LTBP1, BMP1 and NPC2.
- CD109, PSAP, BMP1 and NPC2.
- CD109, IGFBP3, LTBP1, PSAP and BMP1.
- CD109, IGFBP3, LTBP1, PSAP and NPC2.
- CD109, IGFBP3, LTBP1, PSAP, BMP1 and NPC2.

In the particular embodiment when the method comprises determining a risk score, the method further comprises comparing the risk score to a reference value. The term "reference value" has been previously defined. In a preferred embodiment, the reference value according to this embodiment of the invention is obtained from samples of colorectal cancer from one or more patients identified as patients having good prognosis, or from one or more patients identified as patients having bad prognosis, or from a pool of samples of colorectal cancer from patients having good prognosis and patients having bad prognosis. Even more preferably, the reference value is obtained from a pool of samples of colorectal cancer from patients having good prognosis and patients having bad prognosis.

Alternatively, in another particular embodiment, the reference value for the risk score could be based on an individual sample value, such as for example, a value obtained from a sample from the subject being tested, but at an earlier point in time or from a non-cancerous tissue, or can be based on a large number of samples, such as from population of subjects of the chronological age matched group, or based on a pool of samples including or excluding the sample to be tested. In a particular embodiment, the reference value for the risk score can be determined from the expression level of the gene of interest in a non-cancerous colorectal sample. In another particular embodiment, the reference value for the risk score is calculated from the expression level of the gene of interest in a sample of colorectal cancer from one or more patients identified as patients having good prognosis. In another embodiment, the reference value for the risk score can be calculated from an average value of expression level obtained from a pool of non-cancerous colorectal sample. Said reference sample is typically obtained by combining equal amounts of samples from a subject population. In a particular embodiment, the reference value for the risk score is calculated in the same way as the risk score for the patient is calculated but based on a sample or group of samples of reference. For example, if the risk score of the patient is calculated based on the expression levels of certain genes selected from IGFBP3, CD109, LTBP1, PSAP, BMP1 and NPC2, then the reference value is calculated from the expression levels of those same genes in one or more reference sample.

When the risk score is based on the mRNA level of the genes of interest, then the reference value for the risk score should also be obtained from mRNA levels of the same genes of interest. When risk score is based on the level of the proteins encoded by the genes of interest, then the reference value for the risk score should also be obtained from the levels of the proteins encoded by said gene.

For calculating the risk score, the expression levels of the selected genes, preferably the mRNA levels, are normalized as explained before.

To calculate the risk score from colorectal cancer samples from a population of patients with CRC and a good prognosis, the mean and standard deviation of the population is be obtained. The reference value would be calculated as the mean plus the standard deviation.

To calculate the risk score from colorectal samples from a population of patients with CRC and good and poor prognosis, the reference value is obtained using the "optimal cut-off method". This method is based on the log rank analysis and allows obtaining the most significant high-low risk segregation. In this way, the reference value would be the cut-off point that allows separating patients with a good and bad prognosis with greater significance.

The terms "increased" and "decreased" have been previously defined in connection with expression levels of the gene of interest.

*Kit of the invention and uses thereof*

In another aspect the invention relates to a kit comprising reagents adequate for determining the expression levels of the genes CD109, IGFBP3, LTBP1, PSAP, BMP1 and NPC2 and, optionally, reagents for the determining of the expression levels of one or more housekeeping genes, where said reagents specifically bind to the mRNAs or to the polypeptides encoded by said genes.

The terms "expression level", "CD109", "IGFBP3", "LTBP1", "PSAP", "BMP1" and "NPC2" have been previously defined.

The term "housekeeping gene", as used herein, refers to a gene which is generally ubiquitously expressed in all tissues. These genes encode proteins that provide the basic, essential functions that all cells need to survive. Housekeeping genes are usually expressed at the same level in all cells and tissues, but with some variances, especially during cell growth and organism development. Commonly used housekeeping genes include, without limitation, GAPDH (glyceraldehyde-3-phosphate dehydrogenase), beta-actin gene, the tubulin gene, the vinculin gene, genes coding for the 18S or 28S rRNA subunits of the ribosome.

In a preferred embodiment, the reagents adequate for the determination of the expression levels of the genes CD109, IGFBP3, LTBP1, PSAP, BMP1 and NPC2 comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit.

In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes.

In a preferred embodiment, the first component of the kit of the invention comprises a probe which can specifically hybridize to the genes mentioned above.

The term "specifically hybridizing", as used herein, refers to conditions which allow hybridizing of two polynucleotides under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so.

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 $\mu$g/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA.

The microarrays of the invention contain not only specific probes for the polynucleotides indicating a determined pathophysiological situation, but also containing a series of control probes, which can be of three types: normalization controls, expression level controls and hybridization controls.

In the event that the expression levels of the genes according to the present invention is determined by measuring the levels of the polypeptide or polypeptides encoded by said gene or genes, the kits according to the present invention comprise reagents which are capable of specifically binding to said polypeptides.

For this purpose, the arrays of antibodies are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like.

In another aspect, the invention relates to the use of a kit of the invention for predicting the outcome of a patient suffering colorectal cancer, or for determining whether a patient suffering colorectal cancer is candidate to therapy after surgery, or for selecting a patient which is likely to benefit from adjuvant therapy after surgical resection of colorectal cancer. In a preferred embodiment, the use of the kits according to the invention is carried out in patients suffering stage II or stage III CRC.

In another aspect, the invention relates to the use of of the kit of the invention for determining the prognosis of a patient suffering colorectal cancer, or for selecting an adjuvant chemotherapy for a patient suffering colorectal cancer, or for selecting a patient suffering colorectal cancer to receive adjuvant combination chemotherapy.

*Computer implemented method and computer program of the invention*

In another aspect, the invention relates to a computer-implemented method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising:

(i) collecting the data of the expression level of at least one gene selected from the group consisting of CD109, PSAP, NPC2, LTBP1 and BMP1 and, optionally, IGFBP3, in sample from the patient;
(ii) analyzing the collected data by: (a) comparing the expression levels of the genes with a reference value for each gene or (b) calculating a risk score obtained by introducing the expression levels of at least three of said genes in a regression model and comparing the risk score to a reference value; and
(iii) providing the result of the analysis.

The terms "expression level", "CD109", "IGFBP3", "LTBP1", "PSAP", "BMP1", "NPC2", prognosis", "patient", "colorectal cancer", "adjuvant chemotherapy", "adjuvant combination chemotherapy", "reference value", "risk score" and "regression model" have been previously defined.

In a particular embodiment, the risk score is calculated with the formula (I)

$$risk\ score = \Sigma\ (\ \beta i\ x\ Ei)$$

wherein:

$\beta i$ is the regression coefficient of each gene and
Ei is the normalized expression value of each gene.

In a particular embodiment, the regression model is a Cox model and the regression coefficient of each gene is shown in Table 1.

In a particular embodiment, the expression of the genes CD109, PSAP, NPC2, IGFBP3, LTBP1 and BMP1 is determined. In a particular embodiment, the determination of the expression level of the genes is carried out by determining the levels of the corresponding mRNAs or by determining the levels of the polypeptides encoded by said genes.

In a particular embodiment, the sample is a tumor sample, circulating tumor cells or a biofluid.

In a particular embodiment, the patient has stage II or stage III colorectal cancer.

In a particular embodiment, the prognosis is determined as overall survival, disease-specific survival, disease-free survival, distant metastasis-free survival or recurrence.

In a particular embodiment, the adjuvant combination chemotherapy is a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride, and oxaliplatin (FOLFIRINOX). In a particular embodiment, the single-agent adjuvant chemotherapy is 5-fluorouracil (5FU).

In another aspect, the invention relates to a computer program comprising instructions in a computer readable form for implementing the computer-implemented method of the invention.

As will be appreciated by one of skill in the art, the computer readable instructions cause the computing system to carry out the method according to the invention. The device may take the form of an entire hardware embodiment or an embodiment combining software and hardware aspects. Furthermore, the present invention may include a computer program product on a computer-usable storage medium having computer-usable program code means embodied in the medium. Any suitable computer readable medium may be utilized including hard disks, CD-ROMs, optical storage devices, or magnetic storage devices. The computer-usable or computer-readable medium may be or include, for example, but not limited to, an electronic, magnetic, optical, electromagnetic, infrared, or semiconductor system, apparatus, device, or propagation medium.

Computer program code for carrying out operations of the present invention may be written in an object-oriented programming language such as Python, Matlab, JavaE, smalltalk or C++. However, the computer program code for carrying out operations of the present invention may also be written in conventional procedural programming languages, such as the "C" or FORTRAN programming language or even assembly language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

*Methods of treatment of the invention*

In another aspect, the invention refers to a method for treating colorectal cancer in a patient comprising determining the expression level of at least one gene selected from the group consisting of CD109, PSAP, NPC2, LTBP1 and BMP1 in sample from the patient, and comparing the expression level of said gene with a reference value, wherein

- if the expression level of the gene is increased compared to the reference value, the patient is administered adjuvant combination chemotherapy, or
- if the expression level of the gene is decreased compared to the reference value, the patient is administered single-agent adjuvant chemotherapy or no chemotherapy.

In a particular embodiment, the method further comprises determining the expression level of IGFBP3.

In another aspect, the invention relates to a method for treating colorectal cancer in a patient comprising determining in sample from said patient the expression level of at least three genes selected from the group consisting of CD109, PSAP, NPC2, IGFBP3, LTBP1 and BMP1, calculating a risk score obtained by introducing the expression level of said at least three genes in a regression model and comparing the risk score to a reference value, wherein

- if the risk score is increased compared to the reference value, the patient is administered adjuvant combination chemotherapy, or
- if the risk score is decreased compared to the reference value, the patient is administered single-agent adjuvant chemotherapy or no chemotherapy.

The terms "expression level", "CD109", "IGFBP3", "LTBP1", "PSAP", "BMP1", "NPC2", prognosis", "patient", "sample", "colorectal cancer", "adjuvant chemotherapy", "adjuvant combination chemotherapy", "reference value", "increased", "decreased", "risk score" and "regression model" have been previously defined.

In a particular embodiment, the risk score is calculated with the formula (I)

$$risk\ score = \Sigma\ (\ \beta i \times Ei)$$

wherein:

βi is the regression coefficient of each gene and
Ei is the normalized expression value of each gene.

In a particular embodiment, the regression model is a Cox model and the regression coefficient of each gene is shown in Table 1.

In a particular embodiment, the expression of the genes IGFBP3, CD109, LTBP1, PSAP, BMP1 and NPC2 is determined. In a particular embodiment, the determination of the expression level of the genes is carried out by determining the levels of the corresponding mRNAs or by determining the levels of the polypeptides encoded by said genes.

In a particular embodiment, the sample is a tumor sample, circulating tumor cells or a biofluid.

In a particular embodiment, the patient has stage II or stage III colorectal cancer.

In a particular embodiment, the prognosis is determined as overall survival, disease-specific survival, disease-free survival, distant metastasis-free survival or recurrence.

In a particular embodiment, the adjuvant combination chemotherapy is a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride, and oxaliplatin (FOLFIRINOX). In a particular embodiment, the single-agent adjuvant chemotherapy is 5-fluorouracil (5FU).

*Additional aspects of the invention*

1. An *in vitro* method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising determining in a sample from the patient the expression level of at least one gene selected from the group consisting of CD109, PSAP, NPC2, LTBP1 and BMP1.

2. The method according to aspect 1, further comprising determining the expression level of IGFBP3.

3. The method according to any one of aspects 1 or 2, further comprising comparing the expression level of the at least one gene with a reference value, wherein

- if the expression level of the at least one gene is increased compared to the reference value, the patient has a poor prognosis, or adjuvant combination chemotherapy is selected for the patient, or the patient is selected for adjuvant combination chemotherapy, or
- if the expression level of the at least one gene is decreased compared to the reference value, the subject has a good prognosis, or single-agent adjuvant chemotherapy or no adjuvant chemotherapy is selected, or the patient is selected for a single-agent adjuvant chemotherapy or for no adjuvant chemotherapy.

4. The method according to any one of aspects 1 or 2, further comprising

(i) calculating a risk score obtained by introducing the expression level of the at least three genes selected from the group consisting of CD109, PSAP, NPC2, IGFBP3, LTBP1 and BMP1 in a regression model and
(ii) comparing the risk score to a reference value,

wherein

- if the risk score is increased compared to the reference value, the patient has a poor prognosis, or adjuvant combination chemotherapy is selected for the patient, or the patient is selected for adjuvant combination chemotherapy, or
- if the risk score is decreased compared to the reference value, the patient has a good prognosis, or a single-agent adjuvant chemotherapy or no adjuvant chemotherapy is selected, or the patient is selected for a single-agent adjuvant chemotherapy or for no adjuvant chemotherapy.

5. The method according to aspect 4, wherein the risk score is calculated with the formula (I)

$$risk\ score = \Sigma\ (\ \beta i\ x\ Ei)$$

wherein:

βi is the regression coefficient of each gene and
Ei is the normalized expression level of each gene.

6. The method according to aspect 5, wherein the regression model is a Cox model and the regression coefficient of each gene is shown in Table 1.

7. The method according to any one of aspects 1 to 6, wherein the expression of the genes CD109, PSAP, NPC2, IGFBP3, LTBP1 and BMP1 is determined.

8. The method according to any one of aspects 1 to 7, wherein the sample is a tumor sample, circulating tumor cells or a biofluid.

9. The method according to any one of aspects 1 to 8, wherein the patient has stage II or stage III colorectal cancer.

10. The method according to any one of aspects 1 to 9, wherein the prognosis is determined as overall survival, disease-specific survival, disease-free survival, distant metastasis-free survival or recurrence.

11. The method according to any one of aspects 1 to 10, wherein the determination of the expression level of the genes is carried out by determining the levels of the corresponding mRNAs or by determining the levels of the polypeptides encoded by said genes.

12. The method according to any one of aspects 1 to 11, wherein the adjuvant combination chemotherapy is a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride, and oxaliplatin (FOLFIRINOX).

13. The method according to any one of aspects 3 to 12, wherein the single-agent adjuvant chemotherapy is 5-fluorouracil (5FU).

14. A kit comprising reagents adequate for determining the expression levels of the genes CD109, PSAP, NPC2, IGFBP3, LTBP1 and BMP1 and, optionally, reagents for the determining of the expression levels of one or more housekeeping genes, where said reagents specifically bind to the mRNAs or to the polypeptides encoded by said genes.

15. Use of the kit according to aspect 14 for determining the prognosis of a patient suffering colorectal cancer, or for selecting an adjuvant chemotherapy for a patient suffering colorectal cancer, or for selecting a patient suffering colorectal cancer to receive adjuvant combination chemotherapy.

16. The use according to aspect 15, wherein the adjuvant combination chemotherapy is a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride, and oxaliplatin (FOLFIRINOX).

17. A computer-implemented method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising:

(i) collecting the data of the expression level of at least one gene selected from the group consisting of CD109, PSAP, NPC2, IGFBP3, LTBP1 and BMP1 in sample from the patient;
(ii) analyzing the collected data by: (a) comparing the expression levels of the genes with a reference value for each gene or (b) calculating a risk score obtained by introducing the expression levels of at least three of the genes in a regression model and comparing the risk score to a reference value; and
(iii) providing the result of the analysis.

18. A computer program comprising instructions in a computer readable form for implementing the computer-implemented method according to aspect 17.

19. A method for treating colorectal cancer in a patient comprising determining the expression level of at least one gene selected from the group consisting of CD109, PSAP, NPC2, IGFBP3, LTBP1 and BMP1 in sample from the patient, and comparing the expression level of said gene with a reference value, wherein

- if the expression level of the gene is increased compared to the reference value, the patient is administered adjuvant combination chemotherapy, or
- if the expression level of the gene is decreased compared to the reference value, the patient is administered single-agent adjuvant chemotherapy or no adjuvant chemotherapy.

20. A method for treating colorectal cancer in a patient comprising determining in sample from said patient the expression level of at least three genes selected from the group consisting of CD109, PSAP, NPC2, IGFBP3, LTBP1 and BMP1, calculating a risk score obtained by introducing the expression level of said at least three genes in a regression model and comparing the risk score to a reference value, wherein

- if the risk score is increased compared to the reference value, the patient is administered adjuvant combination chemotherapy, or
- if the risk score is decreased compared to the reference value, the patient is administered single-agent adjuvant chemotherapy or no adjuvant chemotherapy.

The invention will be described by way of the following examples which are to be considered as merely illustrative and not limitative of the scope of the invention.

EXAMPLES

MATERIALS AND METHODS

*Cell lines*

KM12SM and KM12L4 cells were obtained from Dr Fidler's lab (MD Anderson Cancer Center. USA). SW620 cells were obtained from the ATCC. Cells were grown in DMEM containing 10% FBS (Invitrogen) and antibiotics at 37 °C in a 5% $CO_2$ humidified atmosphere. Cells were regularly tested for mycoplasma contamination and authenticated by SPR determination.

*Secretome preparation from SW620, KM12SM and KM12L4 cell lines*

To obtain the secretome, cells were seeded at a density of $6 \times 10^6$ cells per plate. Twenty-four hours later, cells were washed with PBS to remove serum and cell debris and cultured with serum-free DMEM for 48 h. Then, medium was collected, centrifuged to remove cell debris and concentrated using Vivaspin devices (Sartorius), at 6000 g for 1 h at 4° C.

*Label-free quantification of secreted proteins*

Concentrated supernatants containing 50 μg of protein were precipitated with 5 vol of acetone overnight at -20 ° C. Next day, the samples were resuspended and cleaned with OMIXs C18. Samples were reconstituted in 5% glycerol and 2% IPG, pH 3.0-10 (GE Healthcare) and loaded onto 12-well isoelectric focusing strips (13 cm, ImmobilineDryStrip, pH 3.0-10) (GE Healthcare) for OFFGEL fractionation. A total of 6 fractions were obtained from each sample and cleaned again with OMIXs before being reconstituted in 5 μL of a 0.1% FA, 2% ACN solution. The peptides were run on a C18-A1 ASY column (Thermo Fisher Scientific) and then eluted on a C18 Biosphere column (10 cm in length, 75 μm in diameter, and 3 μm in particle size). After that, they were separated in a 180 min gradient of 0-35% buffer B in buffer A (buffer A: 0.1% FA / 2% ACN; buffer B: 0.1% FA in ACN) at a flow of 300 nL/min on a nanoEasy HPLC with nanoelectrospray (Proxeon). Mass spectra corresponding to the full scan spectrum (m/z 400-1200) were obtained with a resolution of 60,000 and the 15 most intense ions were selected for fragmentation by collision-induced dissociation (CID) in the ionic trap, with collision energy normalized to 35%. A dynamic exclusion window of 30 s was applied. The tolerance for the parent ion was set at 10 ppm and 0.5 Da for the product ions. Mass spectrometry data acquired on the LTQ-Orbitrap-Velos were analyzed with Maxquant 1.6.15.0 using LFQ quantification (Cox J, et al. Mol Cell Proteomics 2014; 13: 2513-2526). The raw data of the mass spectra (* .raw) corresponding to the cell line experiment were interrogated against the Uniprot human reference proteome UP000005640 (79,038 proteins) database. Statistical and quality control analysis were performed using Perseus 1.6.14 (Tyanova S, et al. Nat Methods 2016; 13: 731-740).

*Microarray analysis of differential gene expression*

For global gene expression analysis, total RNA was isolated from KM12SM and SW620 cells using NucleoSpin RNA kit (Macherey-Nagel). Quality assessment of the RNA was assessed with an Agilent 2100 bio-analyzer. Samples were processed with "GeneChip® WT PLUS Reagent Kit" (Applied Biosystems), hybridized with "Clariom™ S Array, human" (Applied Biosystems) and scanned with a "GeneChip® Scanner 3000 7G" (Applied Biosystems). Raw data were processed with RMA algorithm included in Transcriptome Analysis Console (Applied Biosystems) for normalization and gene level analysis. For each experimental condition, three independent RNA replicates were processed and analyzed. Fold-changes between experimental conditions were calculated as a ratio between the mean of the gene expression signals. Statistical analysis was performed with e-bayes limma included in Transcriptome Analysis Console (Applied Biosystems). Gene expression results were compared with the KM12SM and SW620 expression values from GSE59857 dataset, which contains the gene expression analysis for 155 CRC cell lines (Medico E, et al. Nat Commun 2015; 6: 7002).

*Bioinformatics tools*

The identified and quantified proteins in the proteomic studies were analyzed by systems biology in order to obtain the prediction of the enriched functions. Gene ontology (GO) analysis was performed using g:Profiler web site (Raudvere U, et al. Nucleic Acids Res 2019; 47: W191-W198). Venn diagrams were illustrated using InteractiVenn (Heberle H, et al. BMC Bioinformatics 2015; 16: 169). Unsupervised hierarchical clustering was performed by Euclidean distance method using Perseus 1.6.14. Xena platform was used for visualizing and interpreting cancer genomics data (Goldman MJ, et al. Nat Biotechnol 2020; 38: 675-678).

*Prognostic analyses using public datasets*

Different public gene expression datasets were used for prognosis analysis including datasets from Gene Expression Omnibus (GSE14333 (Jorissen RN, et al. Clin Cancer Res 2009; 15: 7642-7651), GSE17538 (Smith JJ, Gastroenterology 2010; 138: 958-968), GSE39582 (Marisa L, et al. PLoS Med 2013; 10: e1001453) and TCGA Research Network (COADREAD (Muzny DM, et al. Nature 2012; 487: 330-337)). Australian GSE14333 dataset contains clinical and gene expression and disease-free survival data from 290 colorectal cancer patients. GSE17538, GSE39582 and COADREAD databases contain 232 colorectal cancers, 566 colon cancer and 736 colorectal cancer patients, respectively. These cohorts were also used for TNM staging system classification. Predictive value for chemotherapy treatment was evaluated in GSE39582 cohort. In addition, datasets GSE72970 (Del Rio M, et al. Eur J Cancer2017; 76: 68-75) and GSE106584 (Zhu J, et al. Sci Rep 2016; 6: 33273) were used in order to increase the number of patients treated with FOLFIRI and FOLFOX. GSE39582 dataset was also used for information on genome instability and other genetic alterations. CMS subgrouping was performed in GSE14333, GSE39582 and TCGA COADREAD datasets using the "CMSclassifier" R package (Guinney J, et al. Nat Med 2015; 21: 1350-1356). Sadanandam classification of GSE14333 was obtained directly from Sadanandam et al. (Sadanandam A, et al. Nat Med 2013; 19: 619-625). The expression levels for all probes within each sample (patient) were transformed to a z-score value.

*Signature design and risk score development*

Gene selection for the prognostic signature was sequentially performed using GSE39582, TCGA COADREAD and, then, GSE14333 cohorts. Genes with a hazard ratio > 1 in GSE39582 were investigated for a significant log rank p-value applying median cut-off method in the TCGA COADREAD). Then, selected genes were asked to have a significant hazard ratio >1 in GSE14333 for validation. For risk score development, the GSE14333 database was used. Briefly, the risk score for each patient was calculated as the sum of each gene's score, which is derived by multiplying the normalized expression level of each gene by its corresponding coefficient (Risk score = $\Sigma$ Cox coefficient of gene $G_i$ x expression value of gene $G_i$). Then, patients were divided in two groups (i.e. high or low risk) by the optimal cut-off method using X-tile software (Camp RL, et al. Clin Cancer Res 2004; 10: 7252-7259). Gene prognostic signature and risk score classification were validated in GSE39582, TCGA COADREAD and GSE17538 datasets.

*Statistical analysis*

Univariate and multivariate Cox regression analysis were performed using "survival" and "survminer" R packages. Forest plot representation was obtained using "ggplot2" R package. Kaplan-Meier analysis was performed using OriginPro Version 2020 (OriginLab Corporation, Northampton, MA, USA). Significance of gene expression differences between groups was obtained calculating two-sample t-tests for each protein or gene. ANOVA tests were performed in order to detect significant differences in risk score between three or more groups. F statistic and p-value are shown.

RESULTS

*Quantitative label-free proteomic characterization of the metastatic secretome*

The biomarker discovery workflow is detailed in **Fig. 1A.** Protein extracts from concentrated supernatants of each cell line were trypsin-digested and peptides separated in 6 fractions using an OFFGEL instrument. Mass spectrometry results were quantified using MaxQuant LFQ and Perseus. Comparison of the intensity values for each of the quantified proteins among all triplicates and the analysis of the linear correlation between each comparison revealed a high reproducibility of the quantitative data (data not shown). Furthermore, the histograms of the distributions of the LFQ $\log_2$ values of the quantified proteins in each triplicate showed a similar normal distribution pattern, confirming the robustness of the proteomic analysis (data not shown). A principal component analysis (PCA) of the three replicates for each cell line confirmed that KM12SM and L4 cells clustered together while SW620 cells clustered apart (data not shown). In total, 1,570 proteins were identified and 1,564 were quantified in the secretome of the three cell lines (data not shown). Most of the identified and quantified proteins, 1,284 and 884 proteins, respectively, were common to the three cell lines **(Fig. 1B).** However, 260, 70 and 129 proteins were exclusively quantified in the secretome of KM12SM, KM12L4 and SW620, respectively **(Fig. 1B).** Among the quantified proteins, 153 proteins (119 up-regulated and 34 down-regulated) were differentially-secreted between KM12 (L4 and SM) and SW620 cells with a fold-change $\geq 5$ (p-value $\leq 0.05$). Location of the quantified and identified proteins reveals a combination of secreted and cellular proteins likely derived from exosomes and microvesicles. Gene Ontology (GO) analysis of the 119 up-regulated proteins in the KM12 cells showed that extracellular matrix constituent, calcium ion binding, neutrophil degranulation and exocytosis are among the most significantly biological functions. Down-regulated proteins were related with cell migration, including cell polarity, podosome assembly, actin binding and granulocyte activation.

*Gene expression analysis and correlation with proteomic data*

For an initial validation of the expression alterations observed in the secreted proteins of the three cell lines an in-house transcriptomic analysis of KM12SM and SW620 cells (GSE199223) was combined with the publicly-available GSE59857 dataset, which contains the gene expression analysis for 155 CRC cell lines (Medico E, et al. Nat Commun 2015; 6: 7002). Quality control of our transcriptional study indicated a robust Pearson correlation coefficient (data not shown) and normal distribution of the histograms of the signal intensities corresponding to the gene expression (data not shown). An excellent agreement between the transcriptomic analysis and the GSE59857 results was observed, supporting a direct data comparison between both datasets (Fig. 1C). Principal component analysis confirmed a similar clustering of the cell lines by using either our transcriptomic data or the GSE59857 dataset values. Although the overlapping of the proteomic data over the global transcriptomic analysis revealed a weaker match, likely as a consequence of a lower representation of proteins versus identified genes (Fig. 1D), a distribution analysis of the transcriptomic values on the proteomics data indicated an excellent overlap between protein and mRNA alterations for the identified secreted proteins (Fig. 1E). Therefore, gene expression results validated the protein alterations identified in the secretome analysis.

*Discovery, training and validation of prognostic biomarkers*

Then, those genes corresponding to the 119 up-regulated proteins were investigated for their prognostic value according to the REMARK guidelines (McShane LM, et al. J Natl Cancer Inst 2005; 97: 1180-1184). Four different datasets were consecutively used for discovery, training and validation of potential prognostic biomarkers (Fig. 2A). For the initial selection, the CIT cohort (GSE39582, *n* = 566) containing stage and relapse information was used as discovery dataset. Sixty out of 119 genes were found to have a hazard ratio >1, according to the Cox model estimator (Fig. 2A). Training of the 60 genes in the TCGA COADREAD database (n= 736) resulted in the selection of 8 genes with a log rank p-value <0.05 (Fig. 2A). Final validation using the GSE14333 dataset (AUS cohort, n=290 patients) resulted in the selection of six genes (IGFBP3, CD109, LTBP1, PSAP, BMP1 and NPC2) showing a hazard ratio >1 with p-value <0.05 (Fig. 2B, Table 2). Moreover, the six genes consistently showed significant hazard ratios in four datasets (Table 3). As a further validation, the expression of these markers in the metastatic cells and tissues was tested by qPCR, western blot and IHC. Significant differences of gene expression between KM12 and SW620 cells were confirmed by qPCR, with IGFBP3 and PSAP showing the highest and lowest expression, respectively (Fig. 2C). LTBP1, CD109, BMP1, IGFBP3 and NPC2 protein overexpression in the cell supernatants was confirmed by western blot (Fig. 2D).

| Gene symbol | GSE39582 | | COADREAD | | GSE14333 | | GSE17538 | |
|---|---|---|---|---|---|---|---|---|
| | p-value | Hazard ratio | p-value | Log rank | p-value | Hazard ratio | p-value | Hazard ratio |
| CD109 | 0.02 | 1.2956 | 0.01385 | 6.057 | 0 | 1.6524 | 0 | 1.7591 |
| IGFBP3 | 0.0071 | 1.6118 | 0.04106 | 4.174 | 0 | 2.0961 | 0.0069 | 1.8983 |
| NPC2 | 0.1744 | 1.5669 | 0.03759 | 4.323 | 2.00E-04 | 1.4919 | 0.0295 | 2.6606 |
| LTBP1 | 0.0661 | 1.4016 | 0.03585 | 3.767 | 0.0055 | 1.8046 | 0.0013 | 2.8292 |
| BMP1 | 0.1212 | 1.8735 | 0.01062 | 6.527 | 0.01 | 2.1465 | 0.5968 | 1.3771 |
| PSAP | 0.0768 | 1.985 | 0.0379 | 4.31 | 0.0152 | 2.9554 | 0.3445 | 1.4141 |
| THBS2 | 0.0322 | 1.2262 | 0.02348 | 5.133 | 0.264 | 1.5466 | 0.003 | 1.6377 |
| NRP1 | 0.3822 | 1.8482 | 0.00126 | 10.4 | 0.7581 | 1.0455 | 0.5044 | 0.6092 |
| TPP1 | 0.0385 | 3.2131 | 0.0601 | 3.535 | 0.9873 | 0.9967 | 0.7951 | 0.7646 |
| LAMC1 | 5.00E-04 | 2.5411 | 0.6767 | 0.1739 | 0.0024 | 2.0427 | 0.006 | 2.7683 |
| NOV | 0.0319 | 1.9879 | 0.05479 | 3.688 | 0.0024 | 1.6052 | 0.9367 | 0.9687 |
| LAMC2 | 7.00E-04 | 1.8411 | 0.2996 | 1.076 | 0.0842 | 1.5005 | 0.1801 | 1.8588 |
| HSPG2 | 0.0348 | 1.8184 | 0.09374 | 2.809 | 0.3111 | 1.1077 | 0.7663 | 0.8825 |
| HEXA | 0.243 | 1.8079 | 0.9236 | 0.009195 | 0.7553 | 0.9574 | 0.8831 | 1.1413 |
| PTGFRN | 0.0904 | 1.7689 | 0.478 | 0.5035 | 0.9619 | 1.0182 | 0.2404 | 1.787 |
| GANAB | 0.2098 | 1.7495 | 0.08985 | 2.877 | 0.388 | 0.388 | 0.9324 | 1.0438 |
| CTSB | 0.0049 | 1.7402 | 0.5535 | 0.3511 | 0.0043 | 1.9694 | 0.1468 | 1.4419 |
| ALCAM | 0.1004 | 1.6781 | 0.2696 | 1.219 | 0.5062 | 1.1207 | 4.00E-04 | 3.7047 |
| CTSC | 0.0813 | 1.6774 | 0.7392 | 0.1108 | 0.8622 | 1.0493 | 0.117 | 1.7016 |
| GAA | 0.0859 | 1.647 | 0.4783 | 0.5027 | 1.00E-04 | 2.8347 | 0.3413 | 1.4954 |
| PLOD1 | 0.0675 | 1.6371 | 0.698 | 0.1506 | 0.0298 | 1.9182 | 0.0235 | 2.67 |
| GRN | 0.1495 | 1.6027 | 0.6071 | 0.2644 | 0.0623 | 1.7424 | 0.7972 | 0.8884 |
| LAMA5 | 0.0231 | 1.5996 | 0.2923 | 1.109 | 0.0039 | 2.0014 | 0.0113 | 2.6212 |
| PPT1 | 0.1568 | 1.5741 | 0.5064 | 0.4414 | 0.6207 | 1.265 | 0.095 | 2.6454 |
| S100A6 | 0.213 | 1.5644 | 0.7033 | 0.1451 | 0.0048 | 5.1506 | 0.7599 | 0.8382 |
| PDIA3 | 0.246 | 1.5409 | 0.5985 | 0.2773 | 0.6384 | 0.8309 | 0.1216 | 2.1645 |
| ULBP2 | 0.085 | 1.525 | 0.2285 | 1.45 | 0.0323 | 1.3034 | 0.1266 | 2.4218 |
| VEGFA | 0.0962 | 1.5076 | 0.1222 | 2.388 | 0.001 | 2.5416 | 0.012 | 2.5832 |
| PLOD3 | 0.1231 | 1.48 | 0.902 | 0.01515 | 0.5057 | 1.1895 | 0.8977 | 1.0513 |

Table 2. Determination of hazard ratios and long rank p-values for the up-regulated secreted proteins in CRC datasets

**Table 3. Hazard ratios of the 6 selected genes (SEC6) in different datasets**

| | GSE14333 | | GSE17538 | | GSE39582 | | TCGA COADREAD | |
|---|---|---|---|---|---|---|---|---|
| | HR (95% CI) | p-value | HR (95% CI) | p-value | HR (95% CI) | p-value | HR (95% CI) | p-value |
| BMP1 | 2-15 (1.20-3.84) | 0.010 | 1.38 (0.42-451) | 0.597 | 1.87 (0.98-4.86) | 0.121 | 1.24 (0-88-1-75) | 0226 |
| CD109 | 1-65 (1.33-2.05) | 5-3'IE-06 | 1.76 (1.34-2.31) | 4-10E-05 | 1.30 (1.02-1.59) | 0.020 | 1.07(0.94-1 21) | 0.335 |
| IGFBP3 | 2.10 (1.55-2.84) | 1-89E-06 | 1.90 (1.19-3.02) | 0.007 | 1.61 (1.04-2.13) | 0.007 | 1.24 (1-01-1.53) | 0.044 |
| L7BP1 | 1-81 (1.19-2.74) | 0.006 | 2.83 (1.50-3.36) | 0.001 | 1.40 (0.92-1.90) | 0.066 | 1.19 (0.97-1.47) | 0.100 |
| NPC2 | 1.49 (1.21-3.01) | 2.00E-04 | 2.66 (110-6.42) | 0030 | 1.57 (0.89-3.26) | 0.174 | 1.79 (1.11-2.89) | 0.018 |
| PSAP | 2.95 (1.23-7.09) | 0.015 | 2.24 (0.69-7.24) | 0.178 | 1.99 (0.95-4.51) | 0.077 | 1.18 (0.78-1.77) | 0.435 |

*SEC6 risk-score classifier development and validation*

Next, a risk score classifier algorithm (see formula (I)) was developed according to the regression coefficients and normalized expression levels for each of the 6 genes (SEC6) using the GSE14333 dataset, as described in the Methods section.

Formula (I)

$$Risk\ score = \sum_{i=1}^{n} (\beta_i \times E_i)$$

Risk score = 1.0836 * PSAP + 0.7639 * BMP1 + 0.7401 * IGFBP3 + 0.5903 * LTBP1 + 0.5022 * CD109 + 0.4 * NPC2

Patients were divided in two groups (i.e. high or low risk) by the optimal cut-off method using X-tile software (Camp RL, et al. Clin Cancer Res 2004; 10: 7252-7259). An unsupervised hierarchical clustering showed a robust correlation between high expression of the six markers and high risk of the patients as well as a good association between high risk and dead events in the GSE14333 cohort (data not shown) . The risk score was validated using the GSE17538 dataset. The risk score distribution showed that the high risk subset of patients presented poorer survival than the low risk subgroup (Fig. 3A, FIG. 4A). In agreement with these data, patients were correctly stratified according to high and low risk using Kaplan-Meier analyses in both datasets, HR: 2.56 95% CI (1.69-3.87) p-value: 3.67E-6 and HR: 4.33 95% CI (2.16-8.69) p-value: 6.34E-6, respectively (Fig. 3B, Fig. 4B).

Then, the independence of the SEC6 classifier using the GSE14333 and GSE17538 datasets was evaluated (Table 3). In the GSE14333 cohort, the association between SEC6 and other potential risk factors was supervised by univariate and multivariate Cox regression analysis. Stage, chemotherapy and SEC6 expression (high, low) were found to be significant risk factors for disease-free survival (DFS) in univariate analysis, while age, gender and location were not. By multivariate Cox regression analysis, the tumour stage and the SEC6 classifier were an independent risk factor HR: 3,231 95%CI (1.62-6.46) p<0.001 and 3.527 95%CI (1.74-12.44) p value: 4.56E-04, respectively. In a similar way, tumour grade, AJCC stage and risk classification were independent risk factors for overall survival in the GSE17538 cohort in the multivariate analyses (Table 4).

**Table 4. Univariate and multivariate Cox regression analysis of DFS in GSE14333 and OS in GSE17538.**

| | | GSE14333 | | | |
|---|---|---|---|---|---|
| Variable | n | Univariate | | Multivariate | |
| | | HR (95% CI) | p-value | HR (95% CI) | p-value |
| Gender (male or female) | 226 | 0.908 (0.52-1.59) | 0.736 | | |
| Age | 226 | 0.981 (0.96-1.01) | 0.069 | | |
| Location (left, right, rectum) | 226 | 0.831 (0.67-1.11) | 0.223 | | |

(continued)

| GSE14333 | | | | | |
|---|---|---|---|---|---|
| Variable | n | Univariate | | Multivariate | |
| | | HR (95% CI) | p-value | HR (95% CI) | p-value |
| Duke's stage (A, B or C) | 226 | 2.949 (2.03-6.68) | 4.39E-04 | 3.231 (1.62-6.46) | 0.001 |
| Chemotherapy (yes or no) | 226 | 1.892 (1.09-3.30) | 0.025 | 1.189 (0.44-1.60) | 0.597 |
| Risk classification (high risk or low risk) | 226 | 4.334 (2.16-8.86) | 3.49E-05 | 3.527 (1.74-12.44) | 4.56E-04 |

| GSE17538 | | | | | |
|---|---|---|---|---|---|
| Variable | n | Univariate | | Multivariate | |
| | | HR (95% CI) | p-value | HR (95% CI) | p-value |
| Gender (male or female) | 239 | 1.006 (0.67-1.52) | 0.975 | | |
| Age | 239 | 1.008 (0.99-1.02) | 0.304 | | |
| Ethnicity (caucasian or other) | 239 | 0.651 (0.30-1.41) | 0.277 | | |
| Grade (WD, MD or PD) | 239 | 2.339 (1.40-3.92) | 1.27E-03 | 1.807 (1.07-3.04) | 0.026 |
| AJCC stage (I, II or III, IV) | 239 | 3.696 (2.23-6.13) | 3.97E-07 | 3.255 (1.95-5.43) | 6.03E-06 |
| Risk classification (high risk or low risk) | 239 | 2.096 (1.30-3.39) | 2.51E-03 | 1.781 (1.06-2.79) | 0.0283 |

*SEC6 expression correlates with CRC molecular classifiers and specific genetic events*

To explore the association between the SEC6 expression and current CRC subtype classifiers the GSE14333, TCGA COADREAD and GSE39582 cohorts were used. Clustering of the patients according to risk score values revealed a clear association between SEC6 positive expression, high risk prediction and dead events in the three cohorts (data not shown). Moreover, SEC6 positive expression correlated with CMS4 patients that exhibited the highest risk score, followed by CMS1 (Fig. 5A), whereas CMS2 and CMS3 showed the lowest SEC6 expression and risk scores in the three datasets, with p-values 1.02E-12, 9.44E-51 and 7.71E-75, respectively. The highest risk score also correlated with the poor survival-associated stem-like and inflammatory subtypes (Fig. 6A). A combined analysis of the three datasets (n=1534 patients) indicated that most CMS4 patients (75%) were classified as high risk by SEC6 expression (Fig. 6B), whereas CMS2 and CMS3 were mostly classified as low risk (95 and 96%, respectively). Interestingly, CMS1 patients showed an almost 70/30 risk probability according to SEC6 expression (Fig. 6B), suggesting that stratification is highly necessary in this heterogeneous subgroup of patients. Then, the association of the SEC6-based risk prediction with some common genetic events observed in CRC patients (GSE39582 cohort) was explored. The mismatch repair status (p-value=0.018), the CIMP$^+$ status (p-value=0.022) or the presence of BRAF mutations (p-value=0.004) were significantly associated with higher expression of the SEC6 genes (Fig. 5B).These results agree well with previous studies showing a poor prognosis for patients displaying MSI and BRAF-mutations (Tran B, et al. Cancer 2011; 117: 4623-4632). In contrast, mutations in P53 or KRAS, or the chromosomal instability status were not significantly associated with higher risk according to SEC6 expression.

*SEC6 expression associates with poor prognosis in stage II and III patients*

To evaluate the capacity of SEC6 to identify patients with poor prognosis at stage II and III Kaplan-Meier curves were used to compare the survival capacity of SEC6-positive and SEC6-negative subgroups in either a meta-dataset (n=1534 patients) representing combinations of GSE17358, GSE39582 and TCGA COADREAD (Fig. 7) or in each individual cohort (Table 5). Overall survival analysis in the meta-dataset showed the capacity of SEC6 to correctly classify the samples in high and low risk for stage II and III patients. The analysis indicated that the hazard ratio was higher for stage III patients, HR: 2.52, 95% confidence interval (CI) (1.76-3.60) p value: 1.66E-7 than for stage II patients, HR: 1.70, 95% CI (1.15-2.51) p value: 0.00687 (Fig. 7A). Progression-free interval (PFI) was determined using the TCGA COADREAD and indicated a slightly higher hazard ratio for stage II than stage III, HR: 2.41, 95% CI (1.23-4.71) p value: 0.0085 (Stage II) and HR: 1.94, 95% CI (0.96-3.57) p value: 0.05 (Stage III) (Fig. 7B). Finally, disease-specific survival (DSS) prognostic capacity in two cohorts, GSE17538 and TCGA COADREAD, showed equally high hazard ratios for both stages: HR: 4.20, 95% CI (1.49-11.86) p value: 0.0036 (stage II) and HR: 4.25, 95% CI (2.06-8.76) p value: 2.21E-5 (stage III) (Fig. 7C). The results for the individual datasets showed that SEC6-positive tumors were associated with a lower rate of survival and higher recurrence probabilities than SEC6-negative tumors in the three datasets (Table 5). These studies confirm the value of the secreted proteins-based signature in the prediction of patient's outcome at early colorectal cancer stages.

| | | All stages | | Stage I | | Stage II | | Stage III | | Stage IV | |
|---|---|---|---|---|---|---|---|---|---|---|---|
| | | HR (95% CI) | p-value | HR (95% CI) | p-value | HR (95% CI) | p-value | HR (95% CI) | p-value | HR (95% CI) | p-value |
| GSE17538 | OS | 2.10 (1.30-4.08) | 0.00251 | 1.68 (0.23-12.5) | 0.607 | 6.20 (1.97-19.55) | 0.00183 | 2.32 (1.07-5.04) | 0.038 | 1.23 (0.65-2.32) | 0.519 |
| | DSS | 2.42 (1.18-4.96) | 0.0154 | | | 3.26 (0.78-13.68) | 0.106 | 3.95 (1.50-10.40) | 0.00537 | 1.46 (0.62-3.45) | 0.385 |
| TCGA COADREAD | OS | 2.11 (1.41-3.15) | 0.00026 | 9.16 (1.67-50.29) | 0.0108 | 1.54 (0.70-3.50) | 0.3 | 2.83 (1.39-5.76) | 0.00423 | 0.89 (0.39-2.01) | 0.777 |
| | DSS | 2.4 (1.37-4.23) | 0.00232 | | | 4.33 (0.86-21.86) | 0.0756 | 4.44 (1.5-13.1) | 0.007 | 0.77 (0.33-1.82) | 0.557 |
| | PFI | 2.02 (1.39-2.93) | 2.00E-04 | 3.39 (0.57-20.38) | 0.182 | 2.41 (1.23-4.73) | 0.0103 | 1.94 (0.98-3.87) | 0.0587 | 1.07 (0.56-2.05) | 0.828 |
| GSE39582 | OS | 1.99 (1.45-2.73) | 2.08E-05 | | | 1.29 (0.73-2.26) | 0.381 | 2.3 (1.39-3.82) | 0.00117 | 1.19 (0.63-2.26) | 0.592 |
| | RFS | 2.08 (1.5-2.88) | 1.19E-05 | | | 2.22 (1.26-3.94) | 0.00604 | 1.39 (0.84-2.29) | 0.196 | 1.12 (0.57-2.2) | 0.745 |

Table 5. Hazard ratio (95% CI) of high and low risk patients classified by states in survival analyses

*High risk subgroups require aggressive adjuvant chemotherapy*

Finally, the association between SEC6 expression and response to adjuvant chemotherapy for stage II and III patients was explored using the GSE39582 cohort. Most of these patients only received 5-FU combined with calcium folinate (FUFOL), whereas the number of patients that received more aggressive treatments (FOLFIRI or FOLFOX) was much lower. When high and low risk patients were examined together, stage III patients (but not stage II) showed a significant improvement in overall survival after chemotherapy (Fig. 8A). However, when patients were divided in risk subgroups, only SEC6-negative, low risk, stage III patients showed improved survival after FUFOL chemotherapy HR: 0.37, 95%CI (0.2-0.67) p value: 6.45E-4. In contrast, high-risk stage II and III patients did not significantly benefit of the use of FUFOL (Fig. 8B), suggesting that FUFOL is insufficient for the treatment of SEC6-positive, high risk patients. Then, a forest plot was used to determine the hazard ratios in high *versus* low risk subgroups after receiving 5-FU, FUFOL, FOLFOX or FOLFIRI (Fig. 8C). In 5-FU and FUFOL-treated patients, as HR>1 and p-values <0.05, high risk patients showed shorter survival than low risk patients. In FOLFOX and FOLFIRI-treated patients showed lower hazard ratios, suggesting that these treatments were more effective for high risk patients. In summary, only more aggressive therapies are likely to cause increased survival in high risk early stage patients, although low risk patients will benefit of receiving 5-FU-based treatments.

*Each of the genes of the SEC6 signature has predictive value*

Although the most significant predictive value is obtained when combining the SEC6 genes, BMP1, CD109, IGFBP3, LTBP1, NPC2 and PSAP independently show prognostic value too. For each gene, TCGA COADREAD patients were classified in high or low expression using the median expression as cut-off point. Then, Kaplan-Meier analysis was performed to compare the overall survival of high and low expression patients demonstrating that high expression of the selected genes is associated to worse prognosis (Fig. 9). In addition, mRNA levels were also analysed as a continuous variable. Hazard ratio of each gene was calculated by Cox regression model using the mRNA value (Table 6). Disease-free survival was analysed in GSE14333 dataset and overall survival was analysed in GSE17538 and GSE39582 datasets. In most cases, the selected genes were significantly associated to high relative risk.

**Table 6.** Hazard ratio (95% CI) of the selected SEC6 genes in GSE14333 (disease-free survival), GSE17538 and GSE39582 (overall survival) datasets.

|  | GSE14333 | | GSE17538 | | GSE39582 | |
|---|---|---|---|---|---|---|
|  | HR (95% CI) | p-value | HR (95% CI) | p-value | HR (95% CI) | p-value |
| BMP1 | 2.15 (1.20-3.84) | 0.010 | 1.38 (0.42-4.51) | 0.597 | 1.87 (0.98-4.86) | 0.121 |
| CD109 | 1.65 (1.33-2.05) | 5.31E-06 | 1.76 (1.34-2.31) | 4.10E-05 | 1.30 (1.02-1.59) | 0.020 |
| IGFBP3 | 2.10 (1.55-2.84) | 1.89E-06 | 1.90 (1.19-3.02) | 0.007 | 1.61 (1.04-2.13) | 0.007 |
| LTBP1 | 1.81 (1.19-2.74) | 0.006 | 2.83 (1.50-3.36) | 0.001 | 1.40 (0.92-1.90) | 0.066 |
| NPC2 | 1.49 (1.21-3.01) | 2.0OE-04 | 2.66 (1.10-6.42) | 0.030 | 1.57 (0.89-3.26) | 0.174 |
| PSAP | 2.95 (1.23-7.09) | 0.015 | 2.24 (0.69-7.24) | 0.178 | 1.99 (0.95-4.51) | 0.077 |

**Claims**

1. An *in vitro* method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising determining in a sample from the patient the expression level of at least one gene selected from the group consisting of CD109, PSAP, NPC2, LTBP1 and BMP1.

2. The method of claim 1, further comprising determining the expression level of IGFB3.

3. The method according to any one of claims 1 and 2, further comprising comparing the expression level of the at least one gene with a reference value, wherein

    - if the expression level of the at least one gene is increased compared to the reference value, the patient has a poor prognosis, or adjuvant combination chemotherapy is selected for the patient, or the patient is selected for adjuvant combination chemotherapy, or
    - if the expression level of the at least one gene is decreased compared to the reference value, the subject has a good prognosis, or single-agent adjuvant chemotherapy o no adjuvant chemotherapy is selected, or the patient is selected for a single-agent adjuvant chemotherapy or for receiving no adjuvant chemotherapy.

4. The method according to any one of claims 1 or 2, further comprising

(i) calculating a risk score obtained by introducing the expression level of at least three genes selected from the group consisting of CD109, PSAP, NPC2, IGFBP3, LTBP1 and BMP1 in a regression model and
(ii) comparing the risk score to a reference value,

wherein

- if the risk score is increased compared to the reference value, the patient has a poor prognosis, or adjuvant combination chemotherapy is selected for the patient, or the patient is selected for adjuvant combination chemotherapy, or
- if the risk score is decreased compared to the reference value, the patient has a good prognosis, or a single-agent adjuvant chemotherapy or no adjuvant chemotherapy is selected, or the patient is selected for a single-agent adjuvant chemotherapy or for receiving no adjuvant chemotherapy.

5. The method according to claim 4, wherein the risk score is calculated with the formula (I)

$$risk\ score = \Sigma\ (\ \beta i \times Ei)$$

wherein:

$\beta i$ is the regression coefficient of each gene and
$Ei$ is the normalized expression level of each gene.

6. The method according to claim 5, wherein the regression model is a Cox model and the regression coefficient of each gene is shown in Table 1.

7. The method according to any one of claims 1 to 6, wherein the expression of the genes CD109, PSAP, NPC2, IGFBP3, LTBP1 and BMP1 is determined.

8. The method according to any one of claims 1 to 7, wherein the sample is a tumor sample, circulating tumor cells or a biofluid.

9. The method according to any one of claims 1 to 8, wherein the patient has stage II or stage III colorectal cancer.

10. The method according to any one of claims 1 to 9, wherein the prognosis is determined as overall survival, disease-specific survival, disease-free survival, distant metastasis-free survival or recurrence.

11. The method according to any one of claims 1 to 10, wherein the determination of the expression level of the genes is carried out by determining the levels of the corresponding mRNAs or by determining the levels of the polypeptides encoded by said genes.

12. The method according to any one of claims 1 to 11, wherein the adjuvant combination chemotherapy is a combination of folinic acid and 5-fluorouracil (FUFOL), a combination of leucovorin calcium, fluorouracil and irinotecan hydrochloride (FOLFIRI), a combination of leucovorin calcium, fluorouracil and oxaliplatin (FOLFOX) or a combination of leucovorin calcium, fluorouracil, irinotecan hydrochloride, and oxaliplatin (FOLFIRINOX).

13. The method according to any one of claims 3 to 12, wherein the single-agent adjuvant chemotherapy is 5-fluorouracil (5FU).

14. A computer-implemented method for determining the prognosis of a patient suffering colorectal cancer, or for selecting an adjuvant chemotherapy for a patient suffering colorectal cancer, or for selecting a patient suffering colorectal cancer to receive adjuvant combination chemotherapy, the method comprising:

(i) collecting the data of the expression level of at least one gene selected from the group consisting of CD109, PSAP, NPC2, LTBP1 and BMP1 in sample from the patient;
(ii) analyzing the collected data by: (a) comparing the expression levels of the genes with a reference value for each gene or (b) calculating a risk score obtained by introducing the expression levels of at least three genes selected from the group consisting of CD109, PSAP, NPC2, IGFBP3, LTBP1 and BMP1 in a regression model and comparing the risk score to a reference value; and
(iii) providing the result of the analysis.

15. A computer program comprising instructions in a computer readable form for implementing the computer-implemented method according to claim 14.

**A**

FIG. 1

FIG. 1 (CONT.)

**C** Transcriptomics distribution

**D** Proteomics distribution

**E** Transcriptomics distribution

FIG. 1 (CONT.)

**A**

FIG. 2

**B**

**C**

FIG. 2 (CONT.)

**D**

FIG. 2 (CONT.)

**A**

FIG. 3

B

FIG. 3 (CONT.)

A

FIG. 4

B

FIG. 4 (CONT.)

**A**

FIG. 5

B

FIG. 5 (CONT.)

**FIG. 6**

**A**

## Stage II

P = 0.00687
HR = 1.70 (1.15-2.51)

## Stage III

P = 1.6628E-7
HR = 2.52 (1.76-3.60)

FIG. 7

B

## Stage II

## Stage III

**FIG. 7 (CONT.)**

C

## Stage II

## Stage III

**FIG. 7 (CONT.)**

FIG. 8

**FIG. 8 (CONT.)**

**C**  **High _vs_ low risk**

FIG. 8 (CONT.)

FIG. 9

# EUROPEAN SEARCH REPORT

**Application Number**

EP 22 38 2745

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/366835 A1 (POGUE-GEILE KATHERINE LEA [US] ET AL) 24 December 2015 (2015-12-24) * pages 9,10; table 5 * | 1-15 | INV. C12Q1/6886 G01N33/574 |
| A | WO 2020/223233 A1 (GENENTECH INC [US]; HOFFMANN LA ROCHE [CH]) 5 November 2020 (2020-11-05) * claims 1,5; example 1 * | 1-15 | |
| A | YI JOO MI ET AL: "Genomic and Epigenomic Integration Identifies a Prognostic Signature in Colon Cancer", CLINICAL CANCER RESEARCH, vol. 17, no. 6, 15 March 2011 (2011-03-15) , pages 1535-1545, XP093009137, US ISSN: 1078-0432, DOI: 10.1158/1078-0432.CCR-10-2509 Retrieved from the Internet: URL:https://aacrjournals.org/clincancerres /article-pdf/17/6/1535/2003121/1535.pdf> * abstract; figure 4 * * page 1544 * | 1-15 | |

-/--

**TECHNICAL FIELDS SEARCHED (IPC)**

C12Q
G01N

The present search report has been drawn up for all claims

| Place of search | Date of completion of the search | Examiner |
|---|---|---|
| The Hague | 19 December 2022 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 22 38 2745

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KOH HYUN MIN ET AL: "Usefulness of CD109 expression as a prognostic biomarker in patients with cancer : A systematic review and meta-analysis", MEDICINE, vol. 100, no. 11, 1 January 2021 (2021-01-01), page e25006, XP093009045, US ISSN: 0025-7974, DOI: 10.1097/MD.0000000000025006 Retrieved from the Internet: URL:https://www.ncbi.nlm.nih.gov/pmc/artic les/PMC7982172/pdf/medi-100-e25006.pdf> * abstract * * page 6 * ----- | 1-15 | |
| A | ROBLES J ET AL: "Abstract: EACR22-0335 P2-027: The secretome of highly metastatic cells as a source of biomarkers and metastatic effectors in colorectal cancer", EACR 2022, 23 June 2022 (2022-06-23), XP093009257, Seville , Spain Retrieved from the Internet: URL:http://nwm.covr.be/EACR2022abstracts/d ata/HtmlApp/main.html#0> * abstract * ----- | 1-15 | TECHNICAL FIELDS SEARCHED (IPC) |

The present search report has been drawn up for all claims

| Place of search | Date of completion of the search | Examiner |
|---|---|---|
| The Hague | 19 December 2022 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

**EP 22 38 2745**

This annex lists the patent family members relating to the patent documents cited in the above-mentioned European search report.
The members are as contained in the European Patent Office EDP file on
The European Patent Office is in no way liable for these particulars which are merely given for the purpose of information.

**19-12-2022**

| Patent document cited in search report | Publication date | Patent family member(s) | Publication date |
|---|---|---|---|
| US 2015366835 A1 | 24-12-2015 | US 2015366835 A1<br>US 2019076391 A1 | 24-12-2015<br>14-03-2019 |
| WO 2020223233 A1 | 05-11-2020 | NONE | |

EPO FORM P0459

For more details about this annex : see Official Journal of the European Patent Office, No. 12/82

## REFERENCES CITED IN THE DESCRIPTION

### Non-patent literature cited in the description

- **DOWDY ; WEARDEN.** Statistics for Research. John Wiley & Sons, 1983
- AJCC Cancer Staging Manual. Springer, 2002
- **SAMBROOK et al.** Molecular Cloning: A Laboratory Manual. Cold Spring Harbor Press, 1989
- **COX J et al.** *Mol Cell Proteomics,* 2014, vol. 13, 2513-2526
- **TYANOVA S et al.** *Nat Methods,* 2016, vol. 13, 731-740
- **MEDICO E et al.** *Nat Commun,* 2015, vol. 6, 7002
- **RAUDVERE U et al.** *Nucleic Acids Res,* 2019, vol. 47, W191-W198
- **HEBERLE H et al.** *BMC Bioinformatics,* 2015, vol. 16, 169
- **GOLDMAN MJ et al.** *Nat Biotechnol,* 2020, vol. 38, 675-678
- **JORISSEN RN et al.** *Clin Cancer Res,* 2009, vol. 15, 7642-7651
- **SMITH JJ.** *Gastroenterology,* 2010, vol. 138, 958-968
- **MARISA L et al.** *PLoS Med,* 2013, vol. 10, e1001453
- **MUZNY DM et al.** *Nature,* 2012, vol. 487, 330-337
- **DEL RIO M et al.** *Eur J Cancer,* 2017, vol. 76, 68-75
- **ZHU J et al.** *Sci Rep,* 2016, vol. 6, 33273
- **GUINNEY J et al.** *Nat Med,* 2015, vol. 21, 1350-1356
- **SADANANDAM A et al.** *Nat Med,* 2013, vol. 19, 619-625
- **CAMP RL et al.** *Clin Cancer Res,* 2004, vol. 10, 7252-7259
- **MCSHANE LM et al.** *J Natl Cancer Inst,* 2005, vol. 97, 1180-1184
- **TRAN B et al.** *Cancer,* 2011, vol. 117, 4623-4632